# EUROPEAN PATENT APPLICATION

(11) **EP 4 768 040 A1**
(43) Date of publication of application: **01.07.2026**
(21) Application number: 25802490.0
(22) Date of filing: 14.02.2025
(51) Int. Cl.: A61K 38/18, A61P 27/02, A61K 9/08

(54) **COMPOSITION FOR TREATING EYE DISORDERS**

(30) Priority: 13.05.2024 CN 202410590798
(71) Applicant: Wenzhou Medical University, Wenzhou, Zhejiang 325000 (CN); China Resources Zizhu Pharmaceutical Co., Ltd, Beijing 100024 (CN)
(72) Inventor: WANG, Xiaojie, Wenzhou, Zhejiang 325000 (CN); HUI, Qi, Wenzhou, Zhejiang 325000 (CN); LI, Le, Wenzhou, Zhejiang 325000 (CN); YU, Bingjie, Wenzhou, Zhejiang 325000 (CN); DONG, Guojun, Wenzhou, Zhejiang 325000 (CN); SUN, Gang, Wenzhou, Zhejiang 325000 (CN); CHEN, Kaidong, Beijing 100024 (CN); WANG, Yansong, Beijing 100024 (CN); CHEN, Dong, Beijing 100024 (CN); LI, Xiaokun, Beijing 100024 (CN)
(74) Representative: karo IP
(86) International application number: PCT/CN2025/077396
(87) International publication number: WO 2025/236782

(57) **Abstract**

A composition for use in treatment of ocular disorders is provided, comprising human keratinocyte growth factor-2 (hKGF-2) and a stabilizing agent, wherein the stabilizing agent comprises one or more of: 0.1% to 2% (w/v) mannitol or 0.01% to 0.05% (w/v) sodium hyaluronate; 0.1% to 2% (w/v) glycerol; 0.1% to 2% (w/v) trehalose or 0.5% (w/v) human serum albumin; and 0.1% to 2% (w/v) lysine hydrochloride. The compatibility between the hKGF-2 stock solution and pharmaceutical excipients was systematically studied to determine optimal concentration ranges for the stabilizing agent, surfactant, and pH-regulator. Based on these ranges, the denaturation (melting) and aggregation temperatures of hKGF-2 in the solution system were evaluated to establish an optimized formulation, in which the biological activity of hKGF-2 is maintained for an extended period. Compared with existing eye drop formulations, the hKGF-2 eye drops of the present disclosure demonstrate improved efficacy in the treatment of dry eye disease.

## Description

### BACKGROUND OF THE APPLICATION

### 1. Technical Field

The present disclosure generally relates to the field of biomedical technology, and more particularly to a composition for use in treatment of ocular disorders, a product comprising the composition, a method for preparing the composition, and its use in treating ocular disorders.

### 2. Description of Related Art

Dry eye disease refers to a group of ocular surface disorders caused by any abnormality in tear quality or dynamics, leading to tear film instability and subsequent ocular surface lesions. A healthy ocular surface is covered by a tear film structure composed of mucin, aqueous components, and lipids secreted from ocular adnexal glands. The tear film serves to maintain the normal microenvironment of the ocular surface while providing adequate lubrication and protection. When the balance of components in the tear film balance is disrupted and cannot be promptly replenished or repaired, dry eye disease may be induced.

In recent years, the incidence of dry eye disease has gradually increased, and the symptoms tend to worsen with age. Current therapeutic products for alleviating dry eye symptoms include Beifushu^{®} eye drops (manufactured by Zhuhai Essex Bio-Pharmaceutical Co., Ltd.), sodium hyaluronate eye drops, and recombinant bovine basic fibroblast growth factor (rb-bFGF) eye drops. However, these eye drops exhibit limited efficacy in the treatment of dry eye disease and often require combination therapy. WU Cheng et al. studied the clinical efficacy and safety of rb-bFGF eye drops combined with sodium hyaluronate eye drops in the treatment of xerophthalmia. Although the combination therapy improved efficacy to a certain extent, it was only effective in alleviating mild symptoms of dry eye disease. Moreover, given that most dry eye patients are elderly, strict adherence to the combined regimen is difficult, which increases the risk of non-compliance and limits therapeutic effectiveness (WU Cheng, ZHANG Li, LI Wei, "Clinical efficacy and safety of recombinant bovine basic fibroblast growth factor eye drops combined with sodium hyaluronate eye drops in the treatment of xerophthalmia", Chinese Journal of Clinical Rational Drug Use, 2023, 16(36):34-37). Additionally, combined approaches involving common treatment and traditional Chinese medicine have also been explored. However, such treatments are complex, can only provide symptomatic relief, and lack practical applicability (ZHANG Jing, LIU Shaorui, "Observation on the efficacy of comprehensive therapy in the treatment of dry eye syndrome with meibomian gland dysfunction", Laboratory Medicine and Clinic, 2024, 21(2):156-160, 165).

Keratinocyte growth factors (KGFs) belong to the fibroblast growth factor (FGF) family. Keratinocyte growth factor-2 (KGF-2), also known as fibroblast growth factor-10 (FGF-10), is a basic protein growth factor secreted by subcutaneous tissue cells in humans, exhibiting high tissue specificity, favorable stability, and a good safety profile. KGF-2 can stimulate proliferation of both newly generated and senescent epithelial cells. Accordingly, in the field of skincare, it may be used to restore or regenerate damaged tissues and cell metabolism, for example, by promoting skin cell growth and regulating pigment balance, thereby maintaining skin health and delaying aging. Existing studies in nude mice have demonstrated the distinctive developmental effects of KGF-2, including the promotion of hair follicle development and hair growth. Current treatments for dry eye disease are primarily topical and are generally effective only for mild cases, providing temporary symptomatic relief but failing to curative or fundamental therapeutic effects. Patent document CN103656622A discloses an environment-sensitive ocular delivery system containing recombinant human KGF-2 and its applications. The system employs advanced pharmaceutical formulation techniques to prepare KGF-2 silicon plastids, which are further formulated into a smart, environment-sensitive ocular drug delivery system. The preparation process includes at least the steps of: preparing blank silicon plastids, preparing KGF-2-loaded silicon plastids, and preparing a delivery gel. However, the process is complex and requires organic solvents (e.g., chloroform) and preservatives (e.g., ethylparaben), which may pose potential health and environmental risks. Furthermore, the known system cannot be used to treat severe dry eye disease. Therefore, there remains an urgent need for a novel composition that is effective for both mild and moderate-to-severe dry eye disease, providing improved therapeutic efficacy and practical applicability.

Note that, due to potential discrepancies in understanding among those skilled in the art, and the extensive literature and patents reviewed by the applicant during development, not all details are listed due to space constraints. This does not imply that the present disclosure lacks existing art features; rather, it encompasses all relevant existing art features. The applicant reserves the right to supplement this application with further details and features from related existing art, as appropriate, in accordance with relevant regulations.

### SUMMARY OF THE APPLICATION

The present disclosure addresses deficiencies in the existing art and, in one aspect, provides a composition for use in treatment of ocular disorders, comprising human keratinocyte growth factor-2 (hKGF-2) and a stabilizing agent, wherein the stabilizing agent comprises one or more of:
1) 0.1% to 2% (w/v) mannitol or 0.01% to 0.05% (w/v) sodium hyaluronate;
2) 0.1% to 2% (w/v) glycerol;
3) 0.1% to 2% (w/v) trehalose or 0.5% (w/v) human serum albumin; and
4) 0.1% to 2% (w/v) lysine hydrochloride.

According to a preferred embodiment, the concentration of hKGF-2 in the composition of the present disclosure is 50 to 200 µg/mL.

According to a preferred embodiment, the composition of the present disclosure comprises mannitol at a concentration selected from 0.5% or from 1% to 2% (w/v).

Preferably, the composition of the present disclosure comprises mannitol at a concentration of 0.5% (w/v).

More preferably, the composition of the present disclosure comprises mannitol at a concentration of 1.6% (w/v).

According to a preferred embodiment, the composition of the present disclosure comprises glycerol at a concentration of 0.5% to 2% (w/v).

Preferably, the composition of the present disclosure comprises glycerol at a concentration of 1% (w/v).

More preferably, the composition of the present disclosure comprises glycerol at a concentration of 1.5% (w/v).

In another preferred embodiment, the composition of the present disclosure comprises glycerol at a concentration of 0.5% (w/v).

According to a preferred embodiment, the composition of the present disclosure comprises trehalose at a concentration of 0.5% to 2% (w/v).

Preferably, the composition of the present disclosure comprises trehalose at a concentration of 2% (w/v).

According to a preferred embodiment, the composition of the present disclosure comprises lysine hydrochloride at a concentration of 0.1% to 1% (w/v).

Preferably, the composition of the present disclosure comprises lysine hydrochloride at a concentration of 0.1% or 0.5% (w/v).

According to a preferred embodiment, the composition of the present disclosure further comprises a surfactant, wherein the surfactant comprises poloxamer 188 at a concentration of 0.01% to 0.5% (w/v).

Preferably, the surfactant comprises poloxamer 188 at a concentration of 0.01% to 0.1% (w/v).

More preferably, the surfactant comprises poloxamer 188 at a concentration of 0.05% (w/v).

In another preferred embodiment, the surfactant comprises poloxamer 188 at a concentration of 0.01% (w/v).

According to a preferred embodiment, the composition of the present disclosure further comprises a pH regulator comprising citric acid, sodium citrate, or a combination thereof.

According to a preferred embodiment, the composition of the present disclosure further comprises a solvent, which may be water for injection (WFI).

According to a preferred embodiment, the composition of the present disclosure has a pH of 6.0 to 7.0.

Preferably, the composition of the present disclosure has a pH of 6.0 to 6.6.

The present disclosure also provides the use of the composition of the present disclosure for the preparation of a medicament for the treatment of dry eye disease.

The present disclosure further provides the use of the composition of the present disclosure for the preparation of a medicament for the treatment of benzalkonium chloride (BAC)-induced dry eye disease.

The present disclosure further provides the use of the composition of the present disclosure for improving tear film stability, promoting the repair of corneal injury, and/or reducing the severity of conjunctival lesions.

The present disclosure further provides a container comprising the composition of the present disclosure.

Preferably, the container is an eye drop vial.

The present disclosure further provides a kit comprising the container of the present disclosure and instructions and/or a label for the administration and use of the composition of the present disclosure.

The present disclosure further provides a method for preparing a composition for use in treatment of ocular disorders, the method comprising steps of: preparing Solution A comprising glycerol, mannitol, lysine hydrochloride, trehalose, poloxamer 188, citric acid, and sodium citrate; preparing Solution B comprising a prescribed amount of a stock solution of human keratinocyte growth factor-2 (hKGF-2) protein; and mixing Solution A and Solution B to obtain the composition.

The present disclosure further provides a method for preparing a composition for use in treatment of ocular disorders, wherein the method comprises:
adding water for injection (WFI) to a prescribed amount of pharmaceutical excipients and mixing to obtain Solution A;
adding a prescribed amount of human keratinocyte growth factor-2 (hKGF-2) stock solution to a measured amount of WFI and mixing to obtain Solution B; and
mixing Solution A and Solution B to obtain a homogeneous mixture, followed by sterilization,
wherein the pharmaceutical excipients comprise glycerol, mannitol, lysine hydrochloride, trehalose, poloxamer 188, citric acid, and sodium citrate.

According to a preferred mode, the mixing is performed by stirring, wherein the stirring speed is 60 to 90 r/min.

Preferably, the mixing is performed by stirring, wherein the stirring speed is 60 to 80 r/min.

More preferably, the mixing is performed by stirring, wherein the stirring speed is 80 to 90 r/min.

According to a preferred mode, the mixing and stirring time of Solution A is 10 to 30 minutes.

According to a preferred mode, the mixing and stirring time of Solution A and Solution B is 10 to 45 minutes.

The present disclosure further provides a method for treating ocular disorders, the method comprising: administering to a subject in need thereof a pharmaceutical composition comprising a therapeutically effective amount of the composition of the present disclosure or a pharmaceutically acceptable salt thereof, thereby alleviating symptoms of the ocular disorder in the subject.

In some embodiments, the composition for use in treatment of ocular disorders is formulated in a dosage form suitable for a selected route of administration, preferably for ocular administration, and more preferably for topical administration. The composition is suitable for the treatment of corneal epithelial defects, punctate keratopathy, and dry eye syndrome of mild, moderate, or severe degree.

In some embodiments, the dosage of the composition of the present disclosure may be determined by a person skilled in the art. Both single-dose and multiple-dose regimens are contemplated, each having advantages under different clinical conditions.

In some embodiments, the actual amount of the composition of the present disclosure administered for the treatment of ocular disorders may vary based on age, body weight, and condition of the subject, and is determined by the clinical judgment of a medical practitioner or by common dose-escalation studies.

In some embodiments, the composition of the present disclosure is administered ocularly. Specifically, the composition is instilled into the eye 4 to 6 times per day.

For the treatment of ocular disorders mentioned herein, the composition of the present disclosure is preferably administered in the form of eye drops, comprising an active ingredient (hKGF-2) at a concentration ranging from, for example, 0.02% (w/w) to 40% (w/w), in increments of 0.01% (w/w), such as 0.03% (w/w), 0.04% (w/w), or 0.05% (w/w). More preferably, the active ingredient is present at a concentration of 0.02% (w/w) to 30% (w/w). The active ingredient is dissolved in suitable pharmaceutical excipients to obtain an eye drop formulation, which is provided in unit-dose or multi-dose containers, such as eye drop vials or bottles. A unit dose corresponds to the daily dose or a unit sub-dose thereof.

In some embodiments, the active ingredient (hKGF-2), or a pharmaceutical composition comprising hKGF-2, may be administered alone or in combination with one or more therapeutic agents for the treatment or alleviation of dry eye syndrome. The administration may be continued for about 2 to 6 weeks, such as 3, 4, or 5 weeks, or for a longer or shorter duration. The hKGF-2 or the composition comprising hKGF-2 may be administered in an amount of 1 to 2 drops per administration, 4 to 6 times per day.

As used herein, the term "effective amount" refers to an amount of the pharmaceutical composition sufficient to alleviate symptoms and signs associated with ocular disorders, including, but not limited to, reduced basal tear secretion, tear film instability, and pathological damage to the cornea and conjunctiva.

As used herein, the term "subject" refers to a vertebrate, preferably a mammal, and more preferably a human, afflicted with an ocular disorder.

The present disclosure has the following advantages. In one aspect, the present disclosure provides a composition for treating ocular disorders, the composition comprising hKGF-2, a stabilizing agent, a surfactant, a pH regulator, and a solvent. The compatibility between the hKGF-2 stock solution and pharmaceutical excipients was systematically studied to determine preferable concentration ranges of the stabilizing agent, surfactant, and pH regulator. On the basis of the determined ranges, the denaturation (melting) temperature and aggregation temperature of hKGF-2 in the solution system were further evaluated to establish an optimized formulation. The resulting formulation enables long-term stability of the biological activity of hKGF-2.

In another aspect, the present disclosure demonstrates the efficacy of the hKGF-2 eye drops in a benzalkonium chloride (BAC)-induced rabbit model of dry eye syndrome, as assessed by Schirmer I test, tear film break-up time (BUT), corneal fluorescein staining, and histopathological analysis. The results indicate that the hKGF-2 eye drops at low, medium, and high concentrations exhibit favorable therapeutic effects on corneal injury and conjunctival pathology. Furthermore, compared with commercially available eye drops such as Beifushu^{®} eye drops and sodium hyaluronate formulations, the hKGF-2 eye drops provided by the present disclosure show superior efficacy in relieving symptoms of dry eye syndrome.

### BRIEF DESCRIPTION OF THE DRAWINGS

Fig. 1 illustrates the summarized activity values of the formulation at various time points;
Fig. 2 illustrates the summarized peak area values of the formulation at various time points;
Fig. 3 depicts the distribution of melting temperature (Tₘ) values at different levels of the respective excipients;
Fig. 4 depicts the distribution of denaturation onset temperature (Tₒₙₛₑₜ) values at different levels of the respective excipients;
Fig. 5 depicts the distribution of protein aggregation temperatures (T_{agg}266 and T_{agg}473) at different levels of the respective excipients;
Fig. 6 provides histopathological evidence of the effect of human keratinocyte growth factor-2 (hKGF-2) on corneal tissues; and
Fig. 7 provides histopathological evidence of the effect of human keratinocyte growth factor-2 (hKGF-2) on conjunctival tissues.

### DETAILED DESCRIPTION OF THE APPLICATION

The present disclosure will be described in detail with reference to the accompanying drawings. The following embodiments are provided merely to illustrate the present disclosure and are not intended to limit its scope. In the present disclosure, human keratinocyte growth factor-2 eye drops are also referred to as hKGF-2 eye drops. The term "w/v" refers to mass concentration, and the concentration levels described in the following embodiments refer to mass concentration. L-lysine hydrochloride is also referred to herein as lysine hydrochloride. Both lysine hydrochloride and lysine, when used in hKGF-2 eye drops, may produce the corresponding technical effects to varying degrees.

The prescribed amount refers to the weighed mass of an excipient, which is determined by multiplying the preparation volume by the percentage of the excipient in the formulation.

In the present disclosure, the low-concentration group of hKGF-2 (low-dose group) refers to the group of test animals treated with hKGF-2 eye drops at a concentration of 50 µg/ml. The medium-concentration group (medium-dose group) refers to animals treated with eye drops at a concentration of 100 µg/ml, and the high-concentration group (high-dose group) refers to animals treated with eye drops at 200 µg/ml.

The hKGF-2 eye drops are clinically administered by instilling 1 to 2 drops per dose, 4 to 6 times per day. In the present application, the hKGF-2 eye drops may be formulated for packaging in an eye drop vial, and more specifically, may be provided as a preservative-free unit-dose product.

### Embodiment 1

The present embodiment provides a composition for use in treatment of ocular disorders, and more particularly, an eye drop formulation for alleviating dry eye syndrome.

The composition comprises hKGF-2, a stabilizing agent, a surfactant, a pH regulator, and a solvent.

**Table 1: Formulation of hKGF-2 Eye Drops**

| **Ingredient** | **Amount** | **Proportion** | **Excess Addition** | **Function** |
|---|---|---|---|---|
| **hKGF-2** | **80 µg** | **Trace** | **None** | **Active ingredient** |
| **Mannitol** | **2 mg** | **0.5%** | **None** | **Stabilizing Agent** |
| **Glycerol** | **6 mg** | **1.5%** | **None** | **Stabilizing Agent** |
| **Lysine Hydrochloride** | **0.4 mg** | **0.1%** | **None** | **Stabilizing Agent** |
| **Trehalose** | **8 mg** | **2%** | **None** | **Stabilizing Agent** |
| **Poloxamer 188** | **0.2 mg** | **0.05%** | **None** | **Surfactant** |
| **Citric Acid** | **0.05388 mg** | **0.1347‰** | **None** | **pH Regulator** |
| **Sodium Citrate** | **0.962 mg** | **2.405‰** | **None** | **pH Regulator** |
| **Water for Injection** | **382.3841 mg** | **95.596%** | **None** | **Solvent** |

### Embodiment 2

The present embodiment provides a compatibility study between the hKGF-2 stock solution and various excipients.

Specifically, glycerol, trehalose, mannitol, lysine hydrochloride, poloxamer 188, human serum albumin and heparin sodium were selected as stabilizing agents for the formulation of the human keratinocyte growth factor-2 (hKGF-2) ophthalmic solution. The buffer system was composed of 10 mM citrate buffer at pH 6.5, and the protein concentration of hKGF-2 was 0.2 mg/ml. During formulation development, the specific activity of the formulation was screened and optimized by varying the concentrations of individual excipients. A single-variable screening test was conducted to study the compatibility of each excipient with the stock solution across different concentration levels.

After storage at 37 ± 2°C for 7 days, the biological activity was measured. A control sample containing the same protein concentration under identical conditions was used as a reference to calculate the change in hKGF-2 activity. The results are summarized in Table 2.

**Table 2: Specific activity test results of different excipient concentrations**

| **Factor** | | **Biological Activity (IU/ml)** | | | |
|---|---|---|---|---|---|
| **hKGF-2 Stock Solution** | **D0** | **63632** | | | |
| | **D7-37°C** | **27203 (-57.2%)** | | | |
| **Mannitol** | **concentration (w/v)** | **0.1%** | **0.5%** | **1%** | **2%** |
| | **D0** | **46808** | **44638** | **59800** | **39622** |
| | **D7-37°C** | **30688 (-34.4%)** | **36348 (-18.6%)** | **60251 (0.8%)** | **58167 (46.8%)** |
| **Glycerol** | **concentration (w/v)** | **0.1%** | **0.5%** | **1%** | **2%** |
| | **D0** | **60823** | **55687** | **54236** | **67148** |
| | **D7-37°C** | **33088 (-45.6%)** | **33743 (-39.4%)** | **36288 (-33.1%)** | **48903 (-27.2%)** |
| **Trehalose** | **concentration (w/v)** | **0.1%** | **0.5%** | **1%** | **2%** |
| | **D0** | **52645** | **39144** | **52674** | **39593** |
| | **D7-37°C** | **27729 (-47.3%)** | **53724 (37.2%)** | **44237 (-16.0%)** | **44334 (12.0%)** |
| **Lysine Hydrochloride** | **concentration (w/v)** | **0.1%** | **0.5%** | **1%** | **2%** |
| | **D0** | **51933** | **56834** | **74793** | **70641** |
| | **D7-37°C** | **30917 (-40.5%)** | **32720 (-42.4%)** | **42617 (-43.0%)** | **10363 (-85.3%)** |
| **Poloxamer 188** | **concentration (w/v)** | **0.01%** | **0.1%** | **0.5%** | **1%** |
| | **D0** | **51996** | **46277** | **65195** | **50660** |
| | **D7-37°C** | **49765 (-4.3%)** | **58968 (27.4%)** | **45621 (-30.0%)** | **41439 (-18.2%)** |

| | | | | | |
|---|---|---|---|---|---|
| Note: The percentage in parentheses represents the activity change rate (%) = (Dn - D0) / D0 × 100%, where D0 refers to day 0 and D7 refers to day 7. | | | | | |

The results indicate that mannitol, glycerol, trehalose, lysine hydrochloride and poloxamer 188 provide varying degrees of protection to protein activity, demonstrating favorable compatibility with hKGF-2. Accordingly, these excipients are considered suitable for use in the formulation of hKGF-2 eye drops.

### Embodiment 3

The present embodiment provides the process for selecting the pH and buffering system of the hKGF-2 eye drops.

Tears are a slightly opalescent aqueous fluid secreted by the lacrimal glands and conjunctival goblet cells. The primary organic constituents of tears are proteins, including albumin, globulins, lysozyme, and immunoglobulins such as IgA, IgG, and IgE.

The pH of normal human tears ranges between 6.4 and 7.7. Excessive acidity (pH < 4.8) may induce coagulation of mucosal proteins, whereas excessive alkalinity (pH > 9.0) may cause epithelial cell swelling of the ocular mucosa. Both extremes can result in significant ocular irritation, increased tear secretion, and subsequent drug loss, thereby impeding therapeutic efficacy or even causing corneal damage. Considering the ocular tolerance range (pH 4.8 - 9.0), the pH of the hKGF-2 eye drops is preferably selected in the range of 6.0 to 7.0, with a buffering system composed of 10 mM citrate at pH 6.6.

### Embodiment 4

The present embodiment provides the process for selecting the surfactant for the hKGF-2 eye drops.

Surfactants, including anionic and nonionic types, are commonly employed as excipients to enhance protein stability. Specifically, surfactants compete with proteins for the air-liquid interface, thereby reducing protein exposure, and mitigate nonspecific adsorption of proteins to container walls, bottle surfaces, and closures. Furthermore, surfactants increase protein solubility, thereby improving overall stability. Anionic surfactants such as sodium dodecyl sulfate may promote protein denaturation and affect deamidation reactions of food proteins. In contrast, nonionic surfactants such as polysorbates and polyethers are effective in preventing surface adsorption, aggregation, and precipitation of proteins, and in inhibiting their denaturation. In protein-based pharmaceuticals, such as interferon α-2b, G-CSF, and tissue plasminogen activator (tPA) formulations, small amounts of nonionic surfactants such as polysorbate 80 are typically incorporated to inhibit aggregation, likely due to their affinity for air-liquid interfaces, thereby displacing proteins from these interfaces and preventing denaturation. Poloxamer 188, a novel block copolymer comprising polyethylene oxide and polypropylene oxide, is a high-molecular-weight nonionic surfactant. It exhibits good solubility in water and ethanol, partial solubility in anhydrous ethanol, ethyl acetate, and chloroform, and poor solubility in ether and petroleum ether. It possesses slight foaming ability, and a 2.5% aqueous solution has a pH in the range of 5.0 to 7.5. In water for injection, the pH is typically between 6.0 and 7.0. The aqueous solution remains stable in air, while light exposure can lead to a decrease in pH. Poloxamer 188 is stable in both acidic and basic aqueous solutions, as well as in the presence of metal ions.

Accordingly, Poloxamer 188 was selected as the surfactant for the hKGF-2 eye drop formulation in the present embodiment.

### Embodiment 5

The present embodiment provides the process for selecting stabilizing agents for the hKGF-2 eye drops.

Amino acids and sugars were selected to prevent activity loss, degradation, and aggregation of hKGF-2 protein during storage.

Amino acids are commonly used as stabilizing agents in protein formulations to inhibit aggregation, degradation, and oxidation of proteins in solution. Lysine hydrochloride was selected as the stabilizing agent for the hKGF-2 eye drops in this embodiment.

Sugars and polyols are nonspecific protein stabilizers and constitute the most commonly and extensively used class of stabilizing agents. Frequently used examples include sucrose, trehalose, glycerol, mannitol, and sorbitol at concentrations ranging from 1% to 10%. The stabilizing effect of sugars and polyols is closely associated with their concentrations, and the extent of stabilization depends on the specific type of protein. Reducing sugars are avoided due to their potential interaction with amino acids. Polyols such as glycerol and sugars stabilize proteins by selectively solvating them to prevent denaturation. At low concentrations, these additives promote water molecule encapsulation around proteins, thereby excluding hydrophobic agents and enhancing stability. In contrast, high concentrations fail to increase stability and may induce denaturation because a higher proportion of hydrophobic organic solvents begins to affect the proteins. Among various sugars, sucrose and trehalose are most commonly used. Trehalose, a chemically stable disaccharide, is widely employed in the preservation of vaccines, antisera, transfer factors, and bacterial preparations. Owing to its non-reducing nature, high glass transition temperature, and low hygroscopicity, trehalose effectively protects protein structures under extreme conditions such as freezing, heating, hyperosmotic stress, and dehydration. Moreover, exogenous trehalose exhibits non-specific protective effects on biomacromolecules and biological systems.

In the present embodiment, mannitol, glycerol, and trehalose were selected as stabilizing agents in the hKGF-2 eye drop formulation.

### Embodiment 6

The present embodiment provides the selection process for excipient dosages.
1. Eight pharmaceutical excipients, including mannitol, glycerol, trehalose, methionine, lysine hydrochloride, EDTA-2Na, Poloxamer 188, and heparin sodium, were screened at different concentration levels to study their protective effects on hKGF-2 at a concentration of 0.2 mg/ml (200 µg/ml). The specific concentration design is shown in Table 3.

**Table 3: Single-Factor Experimental Design Table**

| **Excipient** | **Level 1** | **Level 2** | **Level 3** | **Level 4** |
|---|---|---|---|---|
| **Mannitol** | **0.1%** | **0.5%** | **1%** | **2%** |
| **Glycerol** | **0.1%** | **0.5%** | **1%** | **2%** |
| **Trehalose** | **0.1%** | **0.5%** | **1%** | **2%** |
| **Methionine** | **0.1%** | **0.5%** | **1%** | **2%** |
| **Lysine Hydrochloride** | **0.1%** | **0.5%** | **1%** | **2%** |
| **EDTA-2Na** | **0.05%** | **0.1%** | **0.5%** | **1%** |
| **Poloxamer 188** | **0.01%** | **0.1%** | **0.3%** | **0.5%** |
| **Heparin Sodium** | **1 µg/ml** | **10 µg/ml** | **100 µg/ml** | **500 µg/ml** |
| **Control** | **hKGF-2 Protein Dilution (200 µg/ml)** | | | |

In Table 3, the concentration levels of mannitol include 0.1%, 0.5%, 1%, and 2%; the concentration levels of glycerol include 0.1%, 0.5%, 1%, and 2%; the concentration levels of trehalose include 0.1%, 0.5%, 1%, and 2%; the concentration levels of methionine include 0.1%, 0.5%, 1%, and 2%; the concentration levels of lysine hydrochloride include 0.1%, 0.5%, 1%, and 2%; the concentration levels of EDTA-2Na include 0.05%, 0.1%, 0.5%, and 1%; the concentration levels of poloxamer 188 include 0.01%, 0.1%, 0.3%, and 0.5%; and the concentration levels of heparin sodium include 1 µg/ml, 10 µg/ml, 100 µg/ml, and 500 µg/ml.

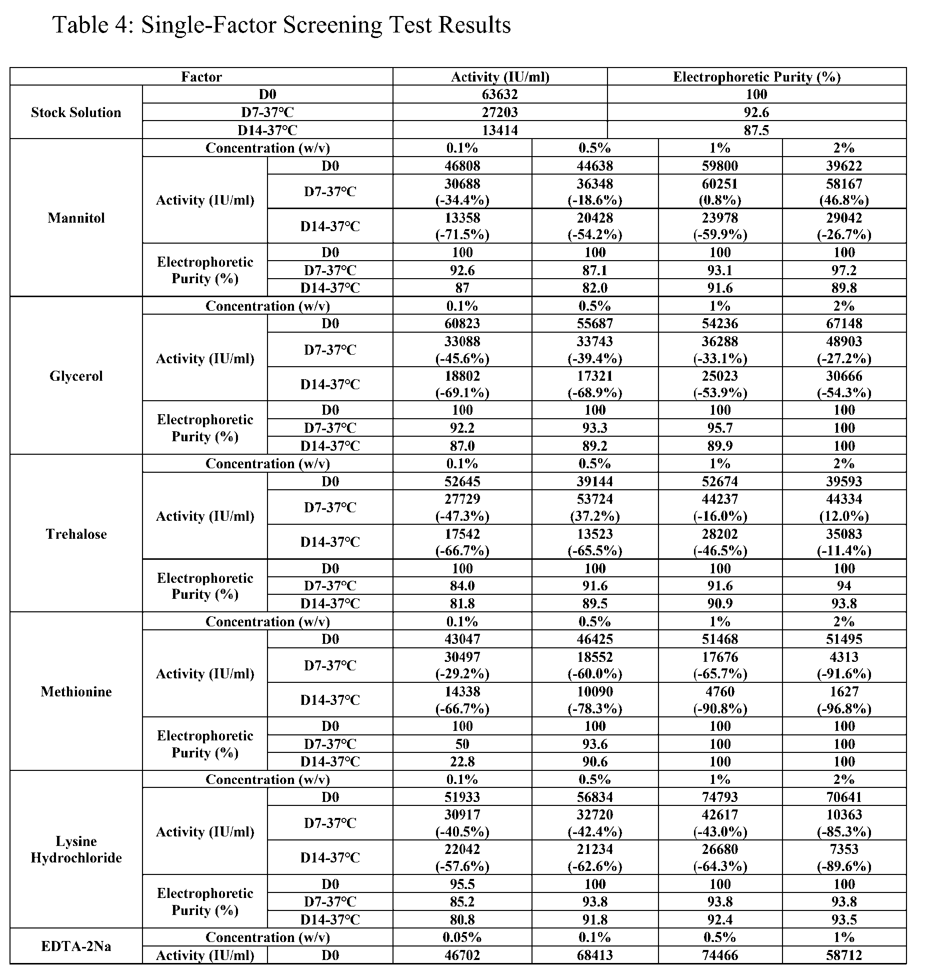

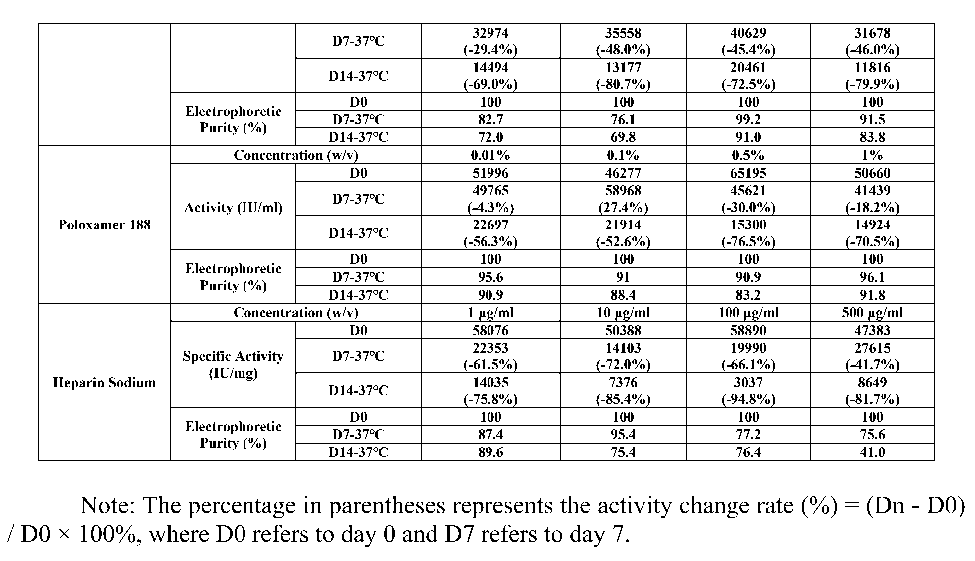

At the same time point, the test groups (mannitol, glycerol, trehalose, methionine, lysine hydrochloride, EDTA-2Na, Poloxamer 188, and heparin sodium) were compared with the control group (stock solution). The test results of activity change rate and electrophoretic purity indicated that methionine, EDTA-2Na, and heparin sodium exhibited no significant protective effect on hKGF-2. In contrast, mannitol, trehalose, Poloxamer 188, and lysine hydrochloride provided certain protection for the protein activity. Mannitol, glycerol, and trehalose exhibited relatively notable and varying degrees of protective effects on the purity of hKGF-2. The preferred concentration ranges were as follows: 0.5% or 1% - 2% for mannitol, 0.5% - 2% for glycerol, 0.5% - 2% for trehalose, 0.5% - 1% for lysine hydrochloride, and 0.01% - 0.1% for Poloxamer 188.

2. In accordance with the single-factor screening results and the principle of orthogonal experimental design, five influencing factors, namely mannitol, glycerol, trehalose, lysine hydrochloride, and Poloxamer 188, were selected. An orthogonal experiment was designed with the five factors at four levels, using the protein activity value as the evaluation metric. The buffer system was a citrate buffer (10 mM, pH 6.5), and the protein concentration of hKGF-2 was 0.2 mg/ml.

**Table 5: Design Table of Different Concentration Levels for Stabilizing Agents and Surfactants**

| **Factor** | **Level 1** | **Level 2** | **Level 3** | **Level 4** |
|---|---|---|---|---|
| **Mannitol** | **1%** | **1.3%** | **1.6%** | **2%** |
| **Glycerol** | **0.5%** | **1%** | **1.5%** | **2%** |
| **Trehalose** | **0.5%** | **1%** | **1.5%** | **2%** |
| **Lysine Hydrochloride** | **0.5%** | **0.6%** | **0.8%** | **1%** |
| **Poloxamer 188** | **0.01%** | **0.05%** | **0.08%** | **0.1%** |

In Table 5, the concentration levels of mannitol include 1%, 1.3%, 1.6%, and 2%; the concentration levels of glycerol include 0.5%, 1%, 1.5%, and 2%; the concentration levels of trehalose include 0.5%, 1%, 1.5%, and 2%; the concentration levels of lysine hydrochloride include 0.5%, 0.6%, 0.8%, and 1%; and the concentration levels of poloxamer 188 include 0.01%, 0.05%, 0.08%, and 0.1%.

**Table 6: Orthogonal Experimental Design Table**

| **Group** | **Mannitol** | **Glycerol** | **Trehalose** | **Lysine Hydrochloride** | **Poloxamer 188** |
|---|---|---|---|---|---|
| **1** | **1** | **1** | **1** | **1** | **1** |
| **2** | **1** | **2** | **2** | **2** | **2** |
| **3** | **1** | **3** | **3** | **3** | **3** |
| **4** | **1** | **4** | **4** | **4** | **4** |
| **5** | **2** | **1** | **2** | **3** | **4** |
| **6** | **2** | **2** | **1** | **4** | **3** |
| **7** | **2** | **3** | **4** | **1** | **2** |
| **8** | **2** | **4** | **3** | **2** | **1** |
| **9** | **3** | **1** | **3** | **4** | **2** |
| **10** | **3** | **2** | **4** | **3** | **1** |
| **11** | **3** | **3** | **1** | **2** | **4** |
| **12** | **3** | **4** | **2** | **1** | **3** |
| **13** | **4** | **1** | **4** | **2** | **3** |
| **14** | **4** | **2** | **3** | **1** | **4** |
| **15** | **4** | **3** | **2** | **4** | **1** |
| **16** | **4** | **4** | **1** | **3** | **2** |

| | | | | | |
|---|---|---|---|---|---|
| Note: In Table 6, each factor level corresponds to the respective concentration level in Table 5, where "1" represents Level 1 in Table 5; "2" represents Level 2; "3" represents Level 3; and "4" represents Level 4. | | | | | |

According to the results of activity change rate and range variance analysis, three optimal formulations were obtained in this embodiment. Specifically, the optimal formulations are: 1.6% mannitol, 1.5% glycerol, 2% trehalose, 0.5% lysine hydrochloride, 0.05% poloxamer 188; 1.6% mannitol, 1% glycerol, 2% trehalose, 0.5% lysine hydrochloride, 0.01% poloxamer 188; and 1.6% mannitol, 0.5% glycerol, 2% trehalose, 0.5% lysine hydrochloride, 0.01% poloxamer 188.

Due to the relatively high osmolality observed in the initially obtained three formulations and the favorable protective effect of 0.5% mannitol on protein activity, the formulations were adjusted as follows: ① 0.5% mannitol, 1% glycerol, 2% trehalose, 0.5% lysine hydrochloride, 0.01% poloxamer 188; ② 0.5% mannitol, 0.5% glycerol, 2% trehalose, 0.5% lysine hydrochloride, 0.01% poloxamer 188; and ③ 0.5% mannitol, 1.5% glycerol, 2% trehalose, 0.5% lysine hydrochloride, 0.05% poloxamer 188.

### Embodiment 7

In the present embodiment, single-factor and multi-factor analyses were combined to facilitate a more comprehensive screening of the optimal eye drop formulation. The specific grouping designs are presented in Tables 7 and 8.
1. In the present embodiment, protein stability of different groups was assessed using the UNcle multifunctional protein stability analysis system (by Unchained Labs) and the UNcle Analysis Software (Version 5.04). The fluorescence and static light scattering (SLS) modules of UNcle were employed to determine the melting (denaturation) temperature and aggregation temperature of hKGF-2 in solution, thereby evaluating its conformational and colloidal stability. In the present embodiment, the DLS (Dynamic Light Scattering) module of the UNcle system was employed to measure the hydrodynamic diameter and polydispersity of proteins in solution at room temperature or a specified temperature, thereby obtaining the particle size and dispersity of the samples. The DLS module continuously monitored changes in the average hydrodynamic diameter and light scattering intensity under thermal stress, which reflect protein denaturation/aggregation. Accordingly, the DLS results serve as an orthogonal validation method for fluorescence-based detection of protein denaturation. The detailed results are presented in Table 9.

Specifically, the tested samples were gently mixed at room temperature and centrifuged. Subsequently, 9 µL of each sample was pipetted into the sample wells, with each sample tested in duplicate for parallel assessment. Two detection modules of the UNcle system were employed, namely full-spectrum fluorescence and static light scattering (SLS), corresponding to the measurement of melting (denaturation) temperature (Tₘ) and aggregation temperature (T_{agg}), respectively.

In the present embodiment, various groups were designed for protein stability analysis, wherein the melting (denaturation) temperature and aggregation temperature of hKGF-2 protein in the solution systems were measured. While validating the original formulations, this embodiment introduced 0.5% human serum albumin as a substitute for trehalose while reducing formulation cost, thereby enhancing the protective effect on protein activity. Additionally, sodium hyaluronate at a concentration of 0.01% to 0.05% was employed as a replacement for mannitol. The buffer system was prepared using 10 mM citrate buffer at pH 6.5, and the protein concentration was 0.2 mg/ml.

### 2. Method for Data Collection and Processing

The temperature ramp program was configured with an initial temperature of 25 °C and an incubation time of 180 seconds, followed by a heating rate of 0.4 °C/min to 95 °C, then continued at 0.6 °C/min with a final hold time of 65 seconds. Fluorescence spectra were acquired over a range of 250 - 720 nm, and data analysis was performed using the barycentric mean (BCM) method. The static light scattering (SLS) module comprises two channels: SLS 266 and SLS 473, corresponding to scattering wavelengths of 266 nm and 473 nm, respectively. SLS 266 is highly sensitive and suitable for low-concentration proteins, primarily reflecting the formation of small aggregates; SLS 473 covers a wider particle size range and is suitable for high-concentration proteins, reflecting the formation of larger aggregates. As the samples in the present embodiment are prone to aggregation, signal responses from both wavelength channels are considered informative.

3. The optimal formulation combination was identified based on the analysis results of protein stability. Details are provided in Table 9.

**Table 7: Single-Factor Experimental Design of Excipients at Different Concentration Levels**

| **Different Concentration Levels of Sodium Hyaluronate** | | | |
|---|---|---|---|
| **Group** | **1** | **2** | **3** |
| **Proportion of Sodium Hyaluronate** | **0.01%** | **0.05%** | **0** |

| **Different Concentration Levels of Glycerol** | | | |
|---|---|---|---|
| **Group** | **4** | **5** | **6** |
| **Proportion of Glycerol** | **0.1%** | **0.5%** | **2%** |

| **Different Concentration Levels of Trehalose** | | | |
|---|---|---|---|
| **Group** | **7** | **8** | **9** |
| **Proportion of Trehalose** | **0.1%** | **0.5%** | **2%** |

| **Different Concentration Levels of Lysine Hydrochloride** | | | |
|---|---|---|---|
| **Group** | **10** | **11** | **12** |
| **Proportion of Lysine Hydrochloride** | **0.1%** | **0.5%** | **2%** |

| **Different Concentration Levels of Poloxamer 188** | | | |
|---|---|---|---|
| **Group** | **13** | **14** | |
| **Proportion of Poloxamer 188** | **0.05%** | **0.1%** | |

| **Different Concentration Levels of Heparin Sodium** | | | |
|---|---|---|---|
| **Group** | **15** | **16** | |
| **Proportion of Heparin Sodium** | **10 µg/ml** | **100 µg/ml** | |

| **Different Concentration Levels of Human Serum Albumin** | | | |
|---|---|---|---|
| **Group** | **17** | **18** | **19** |
| **Proportion of Human Serum Albumin** | **0.01%** | **0.1%** | **0.5%** |

| **Different Concentration Levels of Mannitol** | | | |
|---|---|---|---|
| **Group** | **20** | **21** | **22** |
| **Proportion of Mannitol** | **0.1%** | **0.5%** | **1%** |

Table 7 comprises 22 groups. The concentration levels of sodium hyaluronate were set at 0, 0.01%, and 0.05%; those of glycerol at 0.1%, 0.5%, and 2%; those of trehalose at 0.1%, 0.5%, and 2%; those of lysine hydrochloride at 0.1%, 0.5%, and 2%; those of poloxamer 188 at 0.05% and 0.1%; those of heparin sodium at 10 µg/ml and 100 µg/ml; those of human serum albumin at 0.01%, 0.1%, and 0.5%; and those of mannitol at 0.1%, 0.5%, and 1%.

**Table 8: Multifactorial Design Table of Various Formulations**

| **Group** | **Mannitol** | **Glycerol** | **Trehalose** | **Lysine Hydrochloride** | **Poloxamer 188** | **Human Serum Albumin** |
|---|---|---|---|---|---|---|
| **23** | **0.5%** | **1%** | **0** | **0.5%** | **0.01%** | **0.5%** |
| **24** | **0.5%** | **0.5%** | **0** | **0.5%** | **0.01%** | **0.5%** |
| **25** | **0.5%** | **1.5%** | **0** | **0.5%** | **0.05%** | **0.5%** |
| **26** | **0.5%** | **1%** | **2%** | **0.5%** | **0.01%** | **0** |
| **27** | **0.5%** | **0.5%** | **2%** | **0.5%** | **0.01%** | **0** |
| **28** | **0.5%** | **1.5%** | **2%** | **0.5%** | **0.05%** | **0** |
| **29** | **0.05%** | **1%** | **0** | **0.5%** | **0.01%** | **0.5%** |
| **30** | **0.05%** | **1%** | **2%** | **0.5%** | **0.01%** | **0** |
| **31** | **0.01%** | **1.5%** | **0** | **0.5%** | **0.05%** | **0.5%** |
| **32** | **0.01%** | **1.5%** | **2%** | **0.5%** | **0.05%** | **0** |

| | | | | | | |
|---|---|---|---|---|---|---|
| Note: In Table 8, the groups are sequentially numbered based on Table 7, wherein group numbers 23 to 32 respectively correspond to Formulations 1 to 10. | | | | | | |

**Table 9: Summary of Test Results**

| **Group** | **Mean Tₘ1 (°C)** | **Mean Tₒₙₛₑₜ (°C)** | **Mean Tₘ2 (°C)** | **Mean Tₘ3 (°C)** | **Mean T_{agg} 266 (°C)** | **Mean T_{agg} 473 (°C)** |
|---|---|---|---|---|---|---|
| **3 (Control)** | **57.6** | **52.7** | **/** | **/** | **32.6** | **40.3** |
| **1 (0.01% Sodium Hyaluronate)** | **55.5** | **51.6** | **/** | **/** | **33.2** | **39.5** |
| **2 (0.05% Sodium Hyaluronate)** | **58.7** | **52.1** | **/** | **/** | **32.8** | **35.7** |
| **4 (0.1% Glycerol)** | **57.5** | **51.9** | **/** | **/** | **32.9** | **41.4** |
| **5 (0.5% Glycerol)** | **57.4** | **52.7** | **/** | **/** | **33.6** | **40.6** |
| **6 (2% Glycerol)** | **58.0** | **52.8** | **/** | **/** | **33.8** | **41.6** |
| **7 (0.1% Trehalose)** | **56.8** | **52.4** | **/** | **/** | **32.9** | **41.6** |
| **8 (0.5% Trehalose)** | **56.9** | **52.5** | **/** | **/** | **34.2** | **40.8** |
| **9 (2% Trehalose)** | **57.4** | **53.1** | **/** | **/** | **38.3** | **42.1** |
| **10 (0.1% Lysine Hydrochloride)** | **56.1** | **52.2** | **/** | **/** | **34.6** | **41.6** |
| **11 (0.5% Lysine Hydrochloride)** | **55.4** | **50.5** | **/** | **/** | **32.6** | **40.4** |
| **12 (2% Lysine Hydrochloride)** | **54.2** | **48.1** | **/** | **/** | **37.8** | **38.3** |
| **13 (0.05% Poloxamer 188)** | **57.0** | **53.4** | **/** | **/** | **33.6** | **40.1** |
| **14 (0.1% Poloxamer 188)** | **56.8** | **53.4** | **/** | **/** | **34.2** | **39.7** |
| **15 (10 µg/ml Heparin Sodium)** | **57.0** | **53.4** | **/** | **/** | **35.0** | **41.6** |
| **16 (100 µg/ml Heparin Sodium)** | **60.4** | **55.9** | **/** | **/** | **43.9** | **44.6** |
| **17 (0.01% Human Serum Albumin)** | **56.9** | **52.9** | **/** | **/** | **32.7** | **40.5** |
| **18 (0.1% Human Serum Albumin)** | **54.9** | **51.9** | **/** | **/** | **31.7** | **38.0** |
| **19 (0.5% Human Serum Albumin)** | **53.3** | **49.6** | **/** | **/** | **32.4** | **38.3** |
| **20 (0.1% Mannitol)** | **56.4** | **52.1** | **/** | **/** | **31.2** | **40.2** |
| **21 (0.5% Mannitol)** | **56.6** | **51.8** | **/** | **/** | **33.0** | **39.9** |
| **22 (1% Mannitol)** | **58.0** | **52.6** | **/** | **/** | **32.1** | **40.7** |
| **23 (Formulation 1)** | **50.8** | **48.6** | **84.5** | **/** | **35.7** | **38.3** |
| **24 (Formulation 2)** | **50.6** | **48.7** | **85.6** | **/** | **35.7** | **38.8** |
| **25 (Formulation 3)** | **49.6** | **48.2** | **72.3** | **84.9** | **36.4** | **38.5** |
| **26 (Formulation 4)** | **56.4** | **51.7** | **/** | **/** | **36.2** | **39.0** |
| **27 (Formulation 5)** | **56.9** | **51.8** | **/** | **/** | **36.3** | **40.3** |
| **28 (Formulation 6)** | **57.0** | **51.9** | **/** | **/** | **34.1** | **40.3** |
| **29 (Formulation 7)** | **78.3** | **73.7** | **/** | **/** | **33.5** | **38.5** |
| **30 (Formulation 8)** | **52.0** | **48.9** | **84.8** | **/** | **35.1** | **38.5** |
| **31 (Formulation 9)** | **60.4** | **55.0** | **/** | **/** | **31.4** | **37.4** |
| **32 (Formulation 10)** | **55.3** | **50.2** | **/** | **/** | **34.6** | **38.6** |

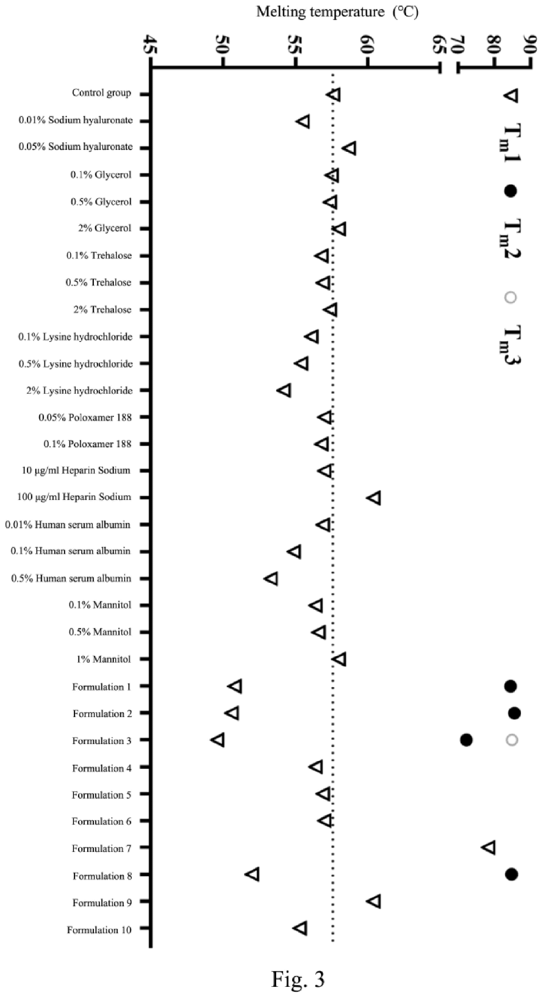

The results are presented in Table 9 and Figs. 3 to 5. Specifically, Fig. 3 illustrates the distribution of Tₘ (melting temperature) values for different levels of each excipient, wherein the horizontal axis represents the excipient types and formulation groups, and the vertical axis represents the corresponding Tₘ values. A higher Tₘ corresponds to enhanced protein stability. Based on the data in Table 9 and Fig. 3, it is demonstrated that higher concentrations of heparin sodium, sodium hyaluronate, glycerol and mannitol contribute to the improvement of the thermal stability of hKGF-2 protein.

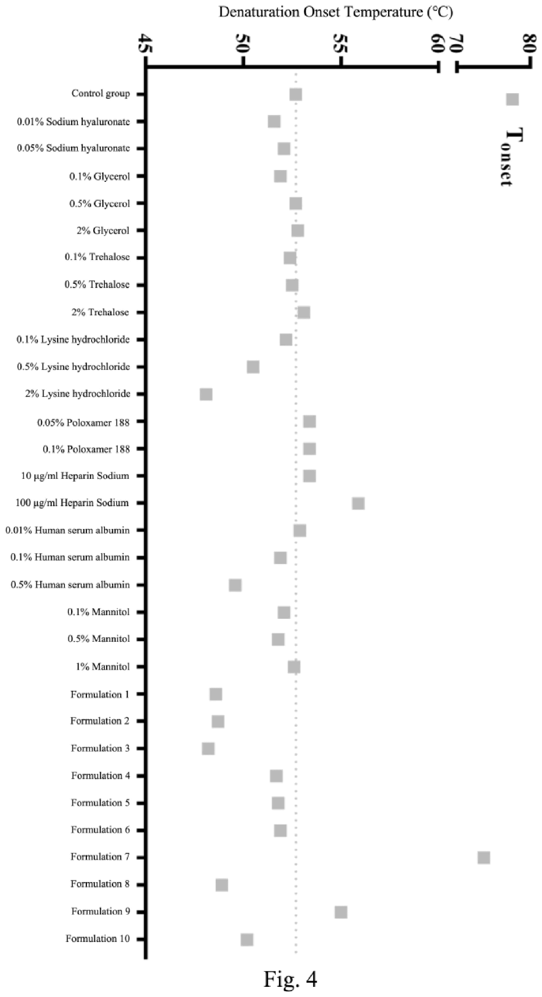

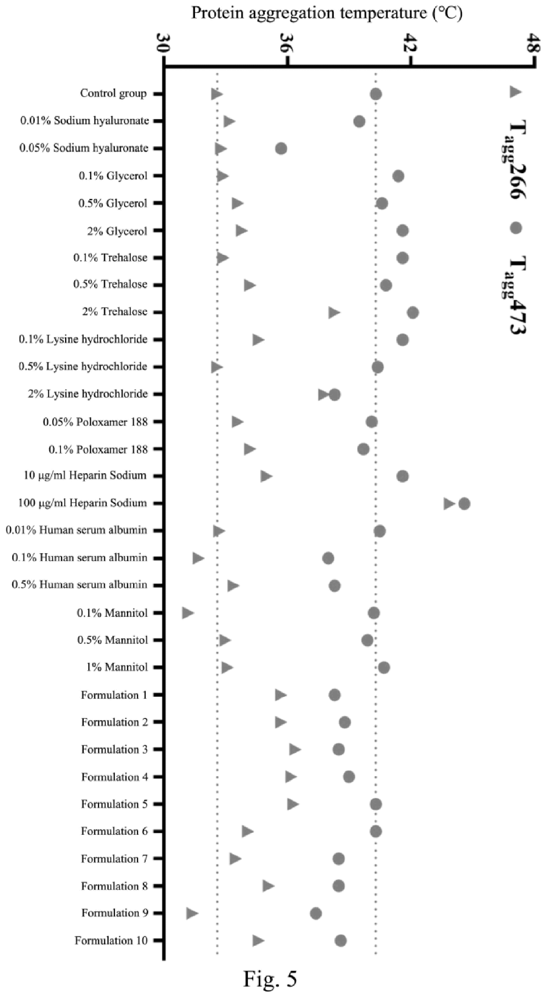

Fig. 4 illustrates the distribution of Tₒₙₛₑₜ (denaturation onset temperature) values corresponding to different levels of excipients and formulations, wherein the horizontal axis denotes the concentration levels of excipients and formulation groups, and the vertical axis denotes the respective Tₒₙₛₑₜ values. Fig. 5 illustrates the distribution of T_{agg}266 and T_{agg}473 (protein aggregation temperatures) for different excipient levels and formulations. The horizontal axis represents the excipient levels and formulation groups, and the vertical axis represents the corresponding aggregation temperatures. T_{agg}266 denotes the aggregation temperature measured at 266 nm, while T_{agg}473 denotes that measured at 473 nm. A higher aggregation temperature corresponds to increased protein stability.

As shown in Fig. 4 and Fig. 5, high concentrations of heparin sodium, glycerol, trehalose, and poloxamer 188, as well as low concentrations of human serum albumin, are effective in enhancing the thermal stability of hKGF-2.

The results demonstrate that the solution systems of Formulations 1 to 10 exhibit high stability. At T_{agg}473, lysine hydrochloride is capable of suppressing protein aggregation; however, the inhibitory effect markedly diminishes as the concentration increases, with 0.1% lysine hydrochloride being preferred. Furthermore, in parallel with validating the original formulations, 0.5% human serum albumin was introduced as a substitute for trehalose, aiming to improve the protective effect on protein activity while reducing overall formulation costs.

### Embodiment 8

The present embodiment provides the study process for validating the stability of the formulations, specifically examining the conformational stability and colloidal stability of hKGF-2 protein in a solution system.

A citrate buffer system (10 mM, pH 6.5) was prepared, and the hKGF-2 protein concentration was set at 0.2 mg/ml. Based on the results of Embodiment 7, Formulations 3, 6, and 9 (as shown in Table 10) were selected from Formulations 1 to 10 for further evaluation of their protective effects on the protein. Accordingly, this embodiment constitutes an optimization process for the formulations provided in Embodiment 7.

The fluorescence and static light scattering (SLS) modules of the UNcle system were utilized to detect the melting (denaturation) temperature and aggregation temperature of the protein in a solution system. In this embodiment, it was observed that lysine hydrochloride suppressed protein aggregation at T_{agg}473, with the inhibitory effect decreasing significantly at higher concentrations. Accordingly, 0.1% lysine hydrochloride was selected as the preferred concentration. Furthermore, while validating the original formulation, 0.5% human serum albumin was introduced in place of trehalose to improve the protective effect on protein activity while reducing formulation cost. In this embodiment, the concentration of lysine hydrochloride in the formulation was reduced to 0.1%.

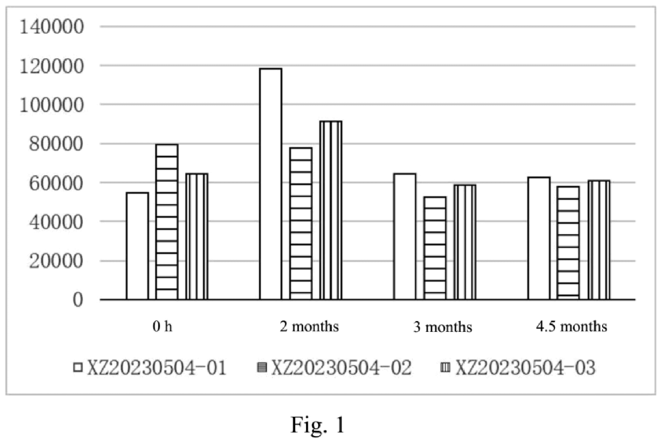

### Detailed experimental results are provided in Figs. 1 - 2 and Table 11.

**Table 10: Formulation Composition Table**

| **Group** | **Mannitol** | **Glycerol** | **Trehalose** | **Lysine Hydrochloride** | **Poloxamer 188** | **Human Serum Albumin** |
|---|---|---|---|---|---|---|
| **Formulation 3** | **0.5%** | **1.5%** | **0** | **0.5%** | **0.05%** | **0.5%** |
| **Formulation 6** | **0.5%** | **1.5%** | **2%** | **0.5%** | **0.05%** | **0** |

| **Group** | **Sodium Hyaluronate** | **Glycerol** | **Trehalose** | **Lysine Hydrochloride** | **Poloxamer 188** | **Human Serum Albumin** |
|---|---|---|---|---|---|---|
| **Formulation 9** | **0.01%** | **1.5%** | **0** | **0.5%** | **0.05%** | **0.5%** |

**Table 11: Summary of Peak Area and Change Rate at Different Time Points for Each Formulation**

| **Time Point (Day)** | **Formulation 3** | **Formulation 6** | **Formulation 9** |
|---|---|---|---|
| **D0** | **374.8** | **446.5** | **428.8** |
| **D7** | **371.5 (-0.9%)** | **416.1 (-6.8%)** | **387.8 (-9.6%)** |
| **D14** | **345.5 (-7.8%)** | **397.4 (-11.0%)** | **375 (-12.5%)** |
| **D21** | **329.6 (-12.1%)** | **395.4 (-11.4%)** | **356.3 (-16.9%)** |
| **D32** | **324.0 (-13.6%)** | **377.3 (-15.5%)** | **338.7 (-21.0%)** |
| **D135** | **322 (-14.1%)** | **438.3 (-1.8%)** | **277.8 (-35.2%)** |
| **D180** | **/** | **451.2 (1.0%)** | |
| **D195** | **/** | **441 (-1.2%)** | |

| | | | |
|---|---|---|---|
| Note: In Figs. 1 and 2, Formulation 3 is labeled as XZ20230504-01, Formulation 6 as XZ20230504-02, and Formulation 9 as XZ20230504-03. | | | |

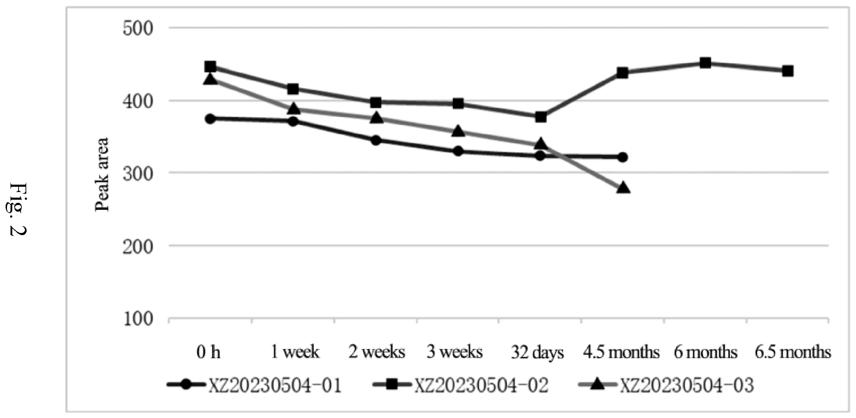

As shown in Fig. 1, Formulation 6 exhibited the lowest activity change rate within 0 to 4.5 months. According to Table 11 and Fig. 2, Formulation 6 maintained the highest biological activity and the lowest activity change rate over 6.5 months. It also showed the largest peak area and the lowest peak area change rate, indicating long-term stability of the hKGF-2 protein. Accordingly, Formulation 6 demonstrated superior overall performance and is recommended for extended evaluation up to 9 months.

Formulation 6, comprising 0.5% mannitol, 1.5% glycerol, 2% trehalose, 0.5% lysine hydrochloride, and 0.05% poloxamer 188, has demonstrated feasibility and is selected for subsequent formulation optimization.

### Embodiment 9

The present embodiment provides the study process regarding the rationality of excipient selection in the formulation.

The hKGF-2 eye drop formulation comprises mannitol, glycerol, trehalose, poloxamer 188, lysine hydrochloride, citric acid, sodium citrate, and water for injection as excipients. A rationale analysis of the excipient selection is provided in Table 12.

**Table 12: Rationale Analysis of Excipients Used in the Eye Drop Formulation**

| **Category** | **Amount** | **Percentage of Total Formulation** | **Within Common Usage Range** | **Suitable for Ocular Administration** | **Functional Impact on Formulation** |
|---|---|---|---|---|---|
| **Mannitol** | **2 mg** | **0.5%** | **Yes** | **Yes** | **Stabilizing Agent** |
| **Glycerol** | **6 mg** | **1.5%** | **Yes** | **Yes** | **Stabilizing Agent** |
| **Lysine Hydrochloride** | **0.4 mg** | **0.1%** | **Yes** | **Yes** | **Stabilizing Agent** |
| **Trehalose** | **8 mg** | **2%** | **Yes** | **Yes** | **Stabilizing Agent** |
| **Poloxamer 188** | **0.2 mg** | **0.05%** | **Yes** | **Yes** | **Surfactant** |
| **Citric Acid** | **0.05388 mg** | **0.1347‰** | **Yes** | **Yes** | **pH Regulator** |
| **Sodium Citrate** | **0.962 mg** | **2.405‰** | **Yes** | **Yes** | **pH Regulator** |
| **Water for Injection** | **382.3841 mg** | **95.596%** | **Yes** | **Yes** | **Solvent** |

Based on the results of the single-factor screening test (Table 4), the preferred concentration ranges are as follows: mannitol, 0.5% or 1% - 2%; glycerol, 0.5% - 2%; trehalose, 0.5% - 2%; lysine hydrochloride, 0.5% - 1%; and poloxamer 188, 0.01% - 0.1%.

Based on the results of the orthogonal tests (Tables 5 and 6), three formulations exhibiting superior activity-preserving effects were initially selected. Following formulation optimization and stability evaluations (Tables 7 - 11, Figs. 1 and 2), the finalized formulation comprises 0.5% mannitol, 1.5% glycerol, 0.1% lysine hydrochloride, 2% trehalose, and 0.05% poloxamer 188.

In a preferred embodiment, the finalized formulation comprises 0.5% mannitol, 1.5% glycerol, 0.1% lysine hydrochloride, 2% trehalose, 0.05% poloxamer 188, 0.1347‰ citric acid, and 2.405‰ sodium citrate.

### Embodiment 10

The present embodiment provides a study into the solvent volume required for dissolving each excipient. When water was used as the solvent, the solubility of mannitol was 18.9 g/100 g at 20°C; lysine hydrochloride, 65 g/100 g at 20°C; trehalose, 68.9 g/100 g at 25°C; citric acid, 59 g/100 g at 20°C; and sodium citrate, 71 g/100 g at 25°C. Poloxamer 188 was readily soluble in water, and glycerol was miscible with water in all proportions.

### 1. Materials and Methods

(1) Prescribed amounts of glycerol, mannitol, lysine hydrochloride, trehalose, poloxamer 188, citric acid, and sodium citrate were weighed and added to 100 ml of water for injection at 15 - 25 °C. The mixture was stirred at 60 - 80 r/min, and the dissolution status was monitored every 5 minutes. Additional water for injection was added as needed until complete dissolution was achieved, thereby obtaining Solution A. The stirring time and total water volume added were recorded.
(2) The remaining amount of water for injection at 15 - 25 °C was measured and mixed. The prescribed amount of protein stock solution was then added to obtain Solution B. The water volume used was recorded, and the appearance of the solution was examined.

**Table 13: Inspection Results of Solution Appearance**

| **Control Item** | **Solution A** | **Solution B** |
|---|---|---|
| **Water for Injection (ml)** | **100** | **Approx. 80** |
| **Stirring Speed (r/min)** | **60 - 80** | **/** |
| **Stirring Time (min)** | **20** | **/** |
| **Appearance** | **Colorless, Clear liquid** | **Colorless, Clear liquid** |

### 2. Results and Analysis

Table 13 summarizes the preparation conditions of each solution. As shown, stirring 100 ml of water for injection at 60 - 80 r/min for more than 20 minutes enabled complete dissolution of the excipients in Solution A, yielding a colorless and clear liquid. Upon adding 80 ml of water for injection to the protein stock solution, Solution B also exhibited a colorless and clear appearance.

### Embodiment 11

The present embodiment provides the study process of the stirring time for Solution A and Solution B.

Given that Solutions A and B are both liquids with approximately a 1:1 volume ratio, low-speed stirring at 60 - 80 r/min was selected based on existing equipment parameters to study the suitable mixing duration for the combined solution.

### 1. Materials and Methods

Solution B was added to Solution A and stirred at 60 - 80 r/min. The appearance and pH of the mixture were monitored every 5 minutes, and osmolality was measured at three time points using four samples collected from different locations.

### 2. Results and Analysis

After Solution A and Solution B were mixed at a stirring speed of 60 - 80 r/min, the CV of osmolality values across four sampling locations was 0.57% at 5 minutes, 0.50% at 10 minutes, and 0.43% at 15 minutes, all below 1%. The pH readings were 6.48, 6.51, and 6.52 at 5, 10, and 15 minutes respectively, indicating stable pH after 5 minutes. The results shown in Tables 14 and 15 (Samples 1 - 4) confirm that uniform distribution was achieved within 5 minutes of stirring.

**Table 14: Mixing Profile of Solution A and Solution B**

| **Stirring Time** | **0 min** | **5 min** | **10 min** | **15 min** |
|---|---|---|---|---|
| **pH** | **6.28** | **6.48** | **6.51** | **6.52** |

**Table 15: Osmolality Values of Samples at Different Stirring Times After Mixing Solution A and Solution B**

| **Stirring Time** | **Osmolality Value (mOsmol/kg)** | | | | **CV Value** |
|---|---|---|---|---|---|
| | **1** | **2** | **3** | **4** | |
| **5 min** | **297** | **301** | **300** | **299** | **0.57%** |
| **10 min** | **299** | **302** | **300** | **302** | **0.50%** |
| **15 min** | **300** | **301** | **299** | **302** | **0.43%** |

### Embodiment 12

The present embodiment provides a study into the filtration rate during sterile filtration after dissolving the hKGF-2 stock solution in other excipients.

### 1. Materials and Methods

In the present embodiment, sterile filtration was performed using Millipore Millex filter units composed of polyethersulfone (PES) membrane, having a diameter of φ33 mm and a pore size of 0.22 µm. Specifically, two 0.22 µm filter units were connected in series to sterilized the bulk solution by filtration. The filtration rate was maintained at no greater than 0.1 L/min, and the filtration process was observed.

### 2. Results and Analysis

When the filtration rate was maintained below 0.1 L/min, the process requirements were satisfied. To ensure control over production time and reduce process risk, the filtration rate for the small-scale trial was set in the range of 0.05 to 0.1 L/min.

### Embodiment 13

### The present embodiment provides a study into the stability of the small-scale process.

According to the process parameters established during initial small-scale optimization, three consecutive small-scale batches were produced under the same conditions. The control parameters across all stages remained generally consistent, thereby verifying the stability of the process. Detailed results are provided in Table 16.

**Table 16: Test Results of Three Consecutive Small-Scale Batches**

| **Process Stage** | **Control Item** | **Batch No.** | | |
|---|---|---|---|---|
| | | **20230602** | **20230603** | **20230604** |
| **Solution Preparation** | **Appearance of Solution A** | **Colorless, Clear liquid** | **Colorless, Clear liquid** | **Colorless, Clear liquid** |
| | **Appearance of Solution B** | **Colorless, Clear liquid** | **Colorless, Clear liquid** | **Colorless, Clear liquid** |
| **Bulk Solution Formation** | **Appearance** | **Colorless, transparent** | **Colorless, transparent** | **Colorless, transparent** |
| | **pH** | **6.3** | **6.3** | **6.3** |
| | **Osmolality** | **350** | **354** | **341** |
| **Sterilization** | **Sterility Test Result** | **Passed** | **Passed** | **Passed** |
| **Yield** | | **298 vials** | **298 vials** | **299 vials** |

Based on the process parameters determined during the small-scale process development, production was performed in accordance with the formulation shown in Table 17. The procedure comprises the following steps:

### 1. Preparation of Solutions

For Solution A, a prescribed amount of pharmaceutical excipients, including glycerol, mannitol, lysine hydrochloride, trehalose, poloxamer 188, citric acid, and sodium citrate, was weighed and added to 60% of the total volume of water for injection maintained at 15 - 25 °C. The mixture was stirred at a speed of 60 - 80 r/min for 30 minutes until completely dissolved.

For Solution B, 30% of the total volume of water for injection at 15 - 25 °C was measured, and the prescribed amount of protein stock solution was added thereto.

Solution B was added to Solution A. The container used for Solution B was rinsed with the remaining 10% of the total water for injection at 15 - 25 °C, and the mixture was stirred at 60 - 80 r/min for 10 minutes.

### 2. Sterilization

The resulting mixed solution was sterile-filtered through two 0.22 µm membrane filters connected in series at a filtration speed of 0.05 - 0.1 L/min to obtain the bulk solution.

### 3. Filling

The bulk solution was filled into low-density polyethylene (LDPE) eye drop vials at a volume of 1 mL per unit.

### 4. Labeling and Storage

Each vial was labeled, inventoried, and stored under refrigeration at 2 - 8 °C.

**Table 17: Composition Table of Materials for Small-Scale Batch Preparation**

| **Material** | **Amount** |
|---|---|
| **hKGF-2** | **60 mg** |
| **Mannitol** | **1.5 g** |
| **Glycerol** | **4.5 g** |
| **Lysine Hydrochloride** | **0.3 g** |
| **Trehalose** | **6 g** |
| **Poloxamer 188** | **0.15 g** |
| **Citric Acid** | **0.0404 g** |
| **Sodium Citrate** | **0.7215 g** |
| **Water for Injection** | **286.7881 g** |

### Embodiment 14

The present embodiment provides a scale-up process based on a proportional enlargement (10 kg) of the small-scale formulation, in order to explore suitable parameters for the solution preparation, sterilization, bulk solution formation, and filling processes in the corresponding equipment.

### 1. Stability of the Solution Preparation Process

### (1) Materials and Methods

① Prescribed amounts of pharmaceutical excipients - including glycerol, mannitol, lysine hydrochloride, trehalose, poloxamer 188, citric acid, and sodium citrate - were weighed and dissolved in 6 kg of water for injection at 20 - 25 °C. The mixture was stirred at a speed of 60 - 80 r/min for 30 minutes.
② Another 6 kg of water for injection at 20 - 25 °C was measured, and the prescribed amount of hKGF-2 stock solution was added. After mixing, Solution B was obtained and its appearance was inspected.

### (2) Results and Analysis

After the solution preparation process was scaled up, the resulting two solutions were consistent with those obtained in the small-scale trial, i.e., both Solution A and Solution B appeared as colorless and clear liquids. These results demonstrate the feasibility of scaling up this process. Detailed results are provided in Table 18.

**Table 18: Appearance of Each Solution**

| **Category** | **Solution A** | **Solution B** |
|---|---|---|
| **Appearance** | **Colorless, Clear liquid** | **Colorless, Clear liquid** |

### 2. Sterilization

In consideration of the increased volume during scale-up, capsule filters were selected as the filtration device. The filter material remained polyethersulfone (PES) membrane, and the filtration flow rate was set to no more than 1 L/min, based on the membrane specification.

### (1) Materials and Methods

The filter unit was manufactured by Pall, with the following specifications: PES membrane, 5-inch size, 0.22 µm pore size.

The bulk solution was sterilized by filtration using two 0.22 µm filters in series. The filtration flow rate did not exceed 1 L/min, and the filtration process was monitored accordingly.

### (2) Results and Analysis

When the filtration flow rate was controlled below 1 L/min, the process met the manufacturing requirements. To minimize process time and reduce associated risks, the optimal flow rate for pilot-scale filtration was determined to be within the range of 0.5 - 1 L/min.

### 3. Bulk Solution Formation Process

### (1) Materials and Methods

① Solution B was added to Solution A and stirred at a speed of 60 - 80 r/min. Samples were collected from four different locations at 5-minute intervals for osmolality and pH measurements, at a total of four time points.

### (2) Results and Analysis

The mixture of Solution A and Solution B remained a clear and transparent solution throughout the stirring process. Stirring was performed at a speed of 80 - 90 r/min. After 5 minutes of stirring, the pH of the solution was 6.41; after 10 minutes, the pH was 6.43; after 15 minutes, the pH was 6.42; and after 20 minutes, the pH was 6.44, indicating that the pH stabilized after 5 minutes of stirring. The CV value for osmolality measurements across different sampling positions was 0.86% at 5 minutes, 0.92% at 10 minutes, 0.41% at 15 minutes, and 0.31% at 20 minutes. Since all CV values were below 1%, the results demonstrate that the solution became uniformly distributed after 5 minutes of stirring and remained stable thereafter. Detailed results are provided in Table 19 and Table 20 (Samples 1 - 4 represent four sampling locations).

**Table 19: Time-dependent pH profiles of the bulk solution**

| **Stirring Time** | **0 min** | **5 min** | **10 min** | **15 min** | **20 min** |
|---|---|---|---|---|---|
| **Appearance** | **Clear, transparent** | **Clear, transparent** | **Clear, transparent** | **Clear, transparent** | **Clear, transparent** |
| **pH** | **6.16** | **6.41** | **6.43** | **6.42** | **6.44** |

**Table 20: Time-dependent osmolality profiles of the bulk solution**

| **Stirring Time** | **Osmolality Value (mOsmol/kg)** | | | | **CV Value** |
|---|---|---|---|---|---|
| | **1** | **2** | **3** | **4** | |
| **5 min** | **308** | **305** | **306** | **311** | **0.86%** |
| **10 min** | **313** | **316** | **318** | **309** | **0.92%** |
| **15 min** | **310** | **313** | **311** | **312** | **0.41%** |
| **20 min** | **312** | **312** | **313** | **314** | **0.31%** |

### 4. Filling Process

### (1) Materials and Methods

The outlet of the preparation tank was connected to a hose, which was further connected to a BT300S peristaltic pump. The pump was set to dispensing mode, and its running speed and duration were adjusted to achieve a filling volume of 10 - 10.5 g. For each parameter combination, 10 samples were prepared. The minimum, maximum, and average values were analyzed to determine the parameter combination capable of stably producing doses within the specified range.

### (2) Results and Analysis

With the peristaltic pump set to dispensing mode, a running speed of 76.8 r/min and a running time of 2 seconds yielded single-dose volumes between 9.9 g and 10.1 g, with an average volume of 10.06 g. This demonstrated the feasibility of producing consistent fill volumes within the specified limits. Detailed results are provided in Table 21.

**Table 21: Filling Conditions under Different Parameter Settings**

| | | | | | | |
|---|---|---|---|---|---|---|
| **Running Speed (r/min)** | **160** | **105** | **78.5** | **77.0** | **76.6** | **76.8** |
| **Running Time (s)** | **1** | **1.5** | **2** | **2** | **2** | **2** |
| **Sample 1** | **9.8762** | **10.0359** | **10.1101** | **10.0169** | **10.0688** | **10.023** |
| **Sample 2** | **9.6955** | **10.0335** | **10.2704** | **9.9357** | **9.9152** | **10.0996** |
| **Sample 3** | **9.2461** | **10.0545** | **10.2611** | **10.0018** | **10.0907** | **10.1407** |
| **Sample 4** | **9.941** | **10.0383** | **10.1951** | **10.0642** | **10.1500** | **10.0703** |
| **Sample 5** | **9.3553** | **9.9684** | **10.3230** | **10.0216** | **10.1106** | **10.0350** |
| **Sample 6** | **9.0988** | **10.0564** | **10.1716** | **9.9788** | **10.0485** | **9.9724** |
| **Sample 7** | **9.7202** | **10.1281** | **10.1385** | **10.0085** | **9.9979** | **10.0564** |
| **Sample 8** | **9.8554** | **10.1286** | **9.7436** | **9.9346** | **9.9547** | **10.1281** |
| **Sample 9** | **9.4468** | **10.1350** | **10.1876** | **10.0545** | **10.1245** | **10.0216** |
| **Sample 10** | **9.385** | **10.0389** | **9.9821** | **9.8837** | **10.0713** | **10.0906** |
| **Maximum Value (g)** | **9.941** | **10.1350** | **10.3230** | **10.0642** | **10.1500** | **10.1407** |
| **Minimum Value (g)** | **9.0988** | **9.9684** | **9.7436** | **9.8837** | **9.9152** | **9.9724** |
| **Average Value (g)** | **9.5620** | **10.0618** | **10.1383** | **9.9900** | **10.0532** | **10.0638** |
| **Remarks** | **High flow rate** | **High flow rate** | **Large variation in values** | **/** | **/** | **/** |

### 5. Pilot Process Confirmation

In accordance with the process parameters determined through pilot-scale studies, three consecutive pilot-scale production batches were conducted. The results of each process control item were generally consistent with those of the lab-scale process in Embodiment 13, thereby demonstrating the stability of the pilot-scale process.

6. Each production step was performed in accordance with the pilot-scale material preparation table, as detailed in Table 22.

**Table 22: Composition Table of Materials for Pilot-Scale Batch Preparation**

| **Material** | **Amount** |
|---|---|
| **hKGF-2** | **2 g** |
| **Mannitol** | **50 g** |
| **Glycerol** | **150 g** |
| **Lysine Hydrochloride** | **10 g** |
| **Trehalose** | **200 g** |
| **Poloxamer 188** | **5 g** |
| **Citric Acid** | **1.347 g** |
| **Sodium Citrate** | **24.05 g** |
| **Water for Injection** | **9559.6003 g** |
| **Batch Size** | **10 kg** |

### (1) Preparation of Solutions

For Solution A, the prescribed amounts of pharmaceutical excipients, including glycerol, mannitol, lysine hydrochloride, trehalose, poloxamer 188, citric acid, and sodium citrate, were weighed and added to 60% of the total volume of water for injection maintained at 15 - 25 °C. The mixture was stirred at a speed of 60 - 80 r/min for 30 minutes until complete dissolution was achieved.

For Solution B, 30% of the total volume of water for injection at 15 - 25 °C was measured, and the prescribed amount of hKGF-2 protein stock solution was added thereto.

Solution B was then added to Solution A. The container of Solution B was rinsed with the remaining 10% of the total water for injection at 15 - 25 °C. The combined solution was stirred at a speed of 60 - 80 r/min for 10 minutes.

### (2) Sterilization

The resulting mixed solution was sterile-filtered through two 0.22 µm membrane filters connected in series. The filtration speed was controlled within the range of 0.5 - 1 L/min to obtain the bulk solution.

### (3) Filling

A peristaltic pump was used for filling, set to operate in dispensing mode with a rotation speed of 76.8 r/min and a running time of 2 seconds. The target fill weight was adjusted to 10 - 10.5 g. Under these conditions, the process enabled consistent production of units within the specified fill weight. The filling speed was 15 - 20 vials per minute.

### (4) Labeling and Storage

Each vial was labeled, inventoried, and stored under refrigeration at 2 - 8 °C.

### 7. Process Changes

The primary changes from small-scale to pilot-scale production involved the use of different equipment, while the relevant process parameters remained largely consistent with those used in the small-scale trial. Details of the changes are shown in Table 23.

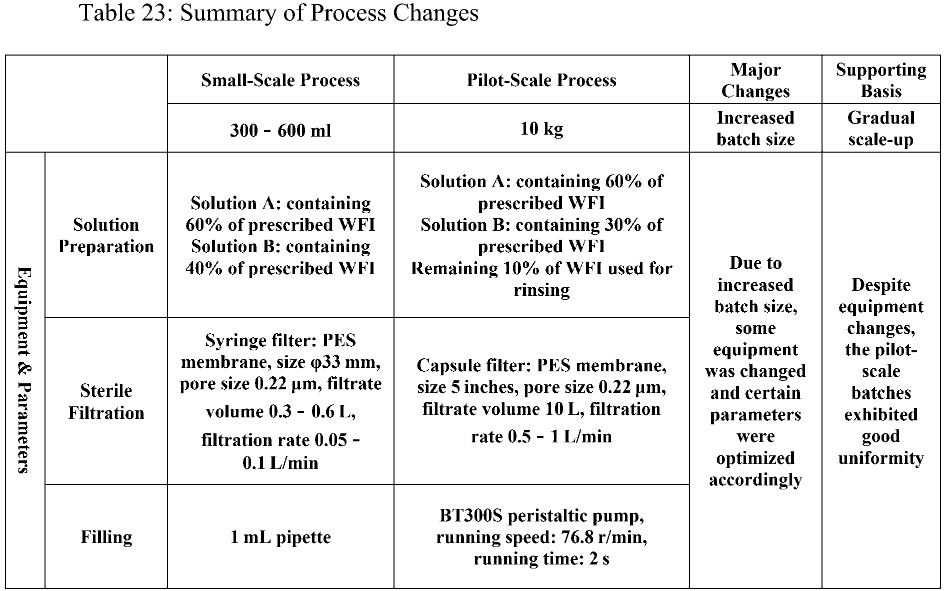

**Table 24: Main Equipment Used in the Production of hKGF-2 Eye Drops**

| **Equipment Name** | **Specification/Model** | **Production Capacity** | **Manufacturer** |
|---|---|---|---|
| **Pulsating Vacuum Sterilizer** | **SGLS-A-650D** | **/** | **Shinva Medical Instrument Co., Ltd.** |
| **Peristaltic Pump** | **BT300S** | **≤20 vials/min** | **Lead Fluid Technology Co., Ltd.** |
| **Electric Stirrer** | **D2010W** | **60 - 1500 r/min** | **Shanghai Meiyingpu Instrument and Meter Manufacturing Co.,Ltd.** |
| **Laminar Flow Cabinet** | **2800*850*600 mm** | **/** | **Yejia Technology (Suzhou Industrial Park) Co., Ltd.** |
| **Automatic Filter Integrity Tester** | **FILGUARD-312** | **/** | **Shanghai Surway Filter Factory** |

### Embodiment 15

The present embodiment provides a batch formulation for hKGF-2 eye drops.

The formulation comprises a stock solution of human keratinocyte growth factor (hKGF-2), mannitol, glycerol, lysine hydrochloride, trehalose, poloxamer 188, citric acid, and sodium citrate. No excess addition of any ingredient was observed. Specific details are presented in Table 25.

**Table 25: Formulation Composition of hKGF-2 Eye drop Batch**

| **Ingredient** | **Amount** | **Excess Addition** | **Standard Implemented** |
|---|---|---|---|
| **hKGF-2** | **2g** | **No** | **Specification for the stock solution (see Section 3.2.S.4.1 of the CTD)** |
| **Mannitol** | **50 g** | **No** | **Chinese Pharmacopoeia, 2020 Edition, Part II** |
| **Glycerol** | **150 g** | **No** | **Chinese Pharmacopoeia, 2020 Edition, Part IV** |
| **Lysine Hydrochloride** | **10 g** | **No** | Chinese Pharmacopoeia, 2020 Edition, Part II |
| **Trehalose** | **200 g** | **No** | Chinese Pharmacopoeia, 2020 Edition, Part IV |
| **Poloxamer 188** | **5 g** | **No** | Chinese Pharmacopoeia, 2020 Edition, Part IV |
| **Citric Acid** | **1.347 g** | **No** | Chinese Pharmacopoeia, 2020 Edition, Part IV |
| **Sodium Citrate** | **24.05 g** | **No** | Chinese Pharmacopoeia, 2020 Edition, Part IV |
| **Water for Injection** | **9559.6003 g** | **No** | Chinese Pharmacopoeia, 2020 Edition, Part II |
| **Total Batch Size** | **10 kg** | **---** | **---** |

According to the sterilization method described in General Chapter 1421 of the Chinese Pharmacopoeia (2020 Edition), the stirring speed is set at 80 - 90 r/min. The mixing time for combining Solution A and Solution B is 45 minutes, and the mixing time for the bulk solution is 20 minutes. The filtration speed for sterile filtration is 0.2 - 0.3 L/min. The bubble point pressure shall be no less than 0.34 MPa. The finished product must pass the sterility test. The filling amount of each unit shall not be less than 93% of the labeled volume, and the average filling amount shall not be less than the labeled volume.

### Embodiment 16

This embodiment provides an experimental study of the effects of the hKGF-2 eye drops described in the preceding embodiments on a rabbit model of dry eye induced by benzalkonium chloride (BAC). A BAC-induced dry eye model was established to evaluate symptoms associated with dry eye in the test animals. In this embodiment, the low-concentration group refers to the group administered with hKGF-2 eye drops at a concentration of 50 µg/ml; the medium-concentration group refers to the group administered with hKGF-2 eye drops at 100 µg/ml; and the high-concentration group refers to the group administered with hKGF-2 eye drops at 200 µg/ml.

### 1. Animal Management

In this embodiment, the test animals were New Zealand white rabbits. The animal housing environment was maintained at a temperature of 16 - 26 °C with a maximum daily temperature fluctuation of no more than 4 °C. Relative humidity was controlled within the range of 40 - 70%, and the ventilation frequency was no less than 8 air changes per hour. Lighting conditions were set to a 12-hour light/12-hour dark cycle (adjustable based on experimental requirements), with an operational illuminance of no less than 200 lx and an animal-level illuminance of 100 - 200 lx. At the start of formal experimentation, the body weight of the rabbits ranged from 2.0 to 2.5 kg. During routine feeding, hay was provided as a dietary supplement when deemed necessary (e.g., in cases of reduced appetite or soft stool). The amount of hay administered was approximately 30 - 50 g per instance, with a corresponding reduction in the amount of standard feed.

In this embodiment, the negative control consisted of a blank vehicle control, while the positive controls included Beifushu^{®} eye drops and sodium hyaluronate eye drops

### 2. Primary Reagents and Preparation Methods

0.1% Benzalkonium Chloride (BAC) Solution: A total of 0.01 g of benzalkonium chloride was weighed and dissolved in 10 mL of sterile normal saline under aseptic conditions in a biosafety cabinet. The resulting solution was aliquoted into 1 mL sterile centrifuge tubes for storage.

1% Sodium Fluorescein Solution: A total of 0.1 g of sodium fluorescein was weighed and dissolved in 10 mL of sterile normal saline under light-protected conditions. The resulting solution was aliquoted into 1 mL sterile centrifuge tubes and stored protected from light.

### 3. Major Instruments

Electronic balances: BS224S, BSA2202S, BL3100, GP2100, and TE601-L (Sartorius Scientific Instruments Systems (Beijing) Co., Limited); Fully automatic autoclave: CLG-40M (ALP Co., Ltd. Japan); Laminar flow clean bench: SW-CJ-2FD (Suzhou Purification Equipment Co., Ltd.);Tissue dehydrator: ASP300S (Leica Biosystems Nussloch GmbH, Germany); Paraffin embedding workstation: EG1160 (Leica Biosystems Nussloch GmbH, Germany); Rotary microtome: RM2235 (Leica Biosystems Nussloch GmbH, Germany); Floating water bath: HI1210 (Leica Biosystems Nussloch GmbH, Germany); Slide dryer: HI1220 (Leica Biosystems Nussloch GmbH, Germany); Image capture units: OLYMPUS DP71 and DP73 (Olympus Corporation, Japan); Pathological image analysis software: IPP6.0 (Media Cybernetics, Inc., USA); cellSens Standard (Olympus Corporation, Japan); ST5020/CV5030 automated staining/coverslipping workstation (Olympus Corporation, Japan).

### 4. Experiment Design

The experiment of the present embodiment was conducted in accordance with Table 26. A total of seven groups were included: a normal control group, a model control group, a low-concentration hKGF-2 group (50 µg/ml), a medium-concentration hKGF-2 group (100 µg/ml), a high-concentration hKGF-2 group (200 µg/ml), a Beifushu^{®} eye drop group, and a sodium hyaluronate eye drop group. After grouping, benzalkonium chloride was administered to the eyes of the test animals to induce the dry eye model. Starting from Day 16 of modeling, drug treatment was carried out at fixed times each day for 21 consecutive days.

### 4.1 Grouping and Modeling Procedures for Test Animals

After the health screening of New Zealand white rabbits was completed, fundus examinations were performed using a slit lamp. Animals with abnormal fundus findings were excluded, and the remaining animals were grouped according to the experimental design. A 0.1% BAC solution was administered to both eyes at 9:00 a.m. and 3:00 p.m. each day for 36 consecutive days to induce dry eye. The normal control group received physiological saline as a negative control. Beginning on Day 16, drug treatment was administered for 21 consecutive days. Based on the dosage design table, both eyes were treated 4 or 5 times per day, with an interval of at least 30 minutes between modeling and drug administration. On Days 0, 7, 15, 19, 23, 26, 30, and 37 (the day after the final treatment), the Schirmer I tear test, tear film break-up time (BUT) test, and corneal fluorescein staining test were conducted. On Day 38 (the second day after the final treatment), the conjunctival and corneal tissues were excised for histopathological examination.

**Table 26: Experimental Grouping and Dosing Protocol for Animals**

| **ID** | **Group** | **Group Size** | **Sex** | **Drug Concentration** | **Dose Volume (µL/eye)** | **Route of Administration** | **Dosing Frequency & Duration** |
|---|---|---|---|---|---|---|---|
| **A** | **Normal control group** | **12** | 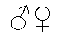 | **NA** | **100** | **Topical ocular instillation** | **4 times/day, for 2 weeks** |
| **B** | **Model control group** | **12** | 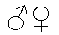 | **NA** | **100** | **Topical ocular instillation** | **4 times/day, for 2 weeks** |
| **C** | **hKGF-2 low-concentration group** | **12** | 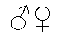 | **50 µg/ml** | **100** | **Topical ocular instillation** | **4 times/day, for 2 weeks** |
| **D** | **hKGF-2 medium-concentration group** | **12** | 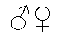 | **100 µg/ml** | **100** | **Topical ocular instillation** | **4 times/day, for 2 weeks** |
| **E** | **hKGF-2 high-concentration group** | **12** | 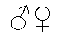 | **200 µg/ml** | **100** | **Topical ocular instillation** | **4 times/day, for 2 weeks** |
| **F** | **Beifushu^{®} eye drop group** | **12** | 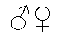 | **4200 IU/ml** | **100** | **Topical ocular instillation** | **4 times/day, for 2 weeks** |
| **G** | **Sodium hyaluronate eye drop group** | **12** | 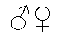 | **1 mg/ml** | **50** | **Topical ocular instillation** | **5 times/day, for 2 weeks** |

| | | | | | | | |
|---|---|---|---|---|---|---|---|
| Note: Each drop of the administered eye drop solution is approximately 50 µL. | | | | | | | |

### 4.2 Evaluation Indicators

All examinations were performed by the same individual under consistent conditions, including examination time, location, illumination intensity, humidity, and temperature. On Days 0, 7, 15, 19, 23, 26, 30, and 37, the rabbits were anesthetized and subjected to the Schirmer I test, tear film break-up time (BUT) test, and corneal fluorescein staining test.
(1) Schirmer I Test: The Schirmer I test was used to evaluate tear secretion in rabbits. The test was performed daily prior to ocular drug administration. On Days 0, 7, 15, 19, 23, 26, 30, and 37 after model induction, the test was conducted at a fixed time. Rabbits were anesthetized by intraperitoneal injection of an appropriate amount of chloral hydrate. A strip of filter paper was placed in the lateral one-third of the conjunctival sac, with the remaining portion hanging outside on the skin surface. After 5 minutes, the length of the wetted area on the strip was measured in millimeters, which represented the tear secretion volume.
(2) Tear Film Break-Up Time (BUT) Test: The BUT test was conducted on Days 0, 7, 15, 19, 23, 26, 30, and 37. Rabbits were anesthetized by intraperitoneal injection of an appropriate amount of chloral hydrate. After immobilization, 1% fluorescein sodium solution was applied to the lower conjunctival sac using a glass rod. Once the eyelids were closed and the dye was evenly distributed over the corneal surface, the eyelids were gently opened to expose the cornea. Under a slit lamp equipped with cobalt blue light, the time from the last blink to the appearance of the first break in the tear film was recorded. Each eye was tested three times, and the average value was calculated and recorded as the BUT for that day.
(3) Corneal Fluorescein Staining Test: A glass rod dipped in 1% fluorescein sodium solution was used to apply the dye to the lower conjunctival sac. After 1 minute, corneal staining was observed and photographed under a slit lamp microscope. The corneoconjunctival region was divided into four quadrants: the cornea, the temporal conjunctiva, the inferior bulbar conjunctiva, and the superior bulbar conjunctiva. Each quadrant was graded on a scale of 0 to 3 based on the staining density and area, where 0 indicated no staining; 1 indicated sparse punctate staining; 2 indicated dense punctate staining; and 3 indicated filamentous, patchy, or block staining. The total score ranged from 0 to 12. A total score of less than 3 was considered negative; 3 to 6 was weakly positive; 6 to 9 was positive; and 9 to 12 was strongly positive. The scores of both eyes were summed and analyzed. All fluorescein staining evaluations were conducted in a blinded manner by the same observer.
(4) Histopathological Examination of Lacrimal Gland and Cornea

On Day 38, the rabbits were euthanized, and the conjunctival and corneal tissues were excised and fixed in formalin. The specimens were subjected to hematoxylin and eosin (HE) staining and examined histologically under a light microscope.

### 5. Statistical Analysis Method

All data were analyzed using GraphPad Prism. The results were expressed as mean ± standard deviation (SD), and one-way analysis of variance (ANOVA) was used for statistical comparisons between groups.

### 6. Experiment Results

6.1 The effects of hKGF-2 on basal tear secretion in rabbits (Schirmer I tear test) are shown in Table 27.

**Table 27: Schirmer I Test Results Before and After Treatment in Each Group (mm) (x ±s)**

| **Group** | **D0** | **D7** | **D15** | **D19** | **D23** | **D26** | **D30** | **D37** |
|---|---|---|---|---|---|---|---|---|
| **Normal control group** | **15.92±5.20** | **16.38±5.25** | **15.96±5.62** | **15.67±3.56** | **16.25±4.18** | **16.79±3.56** | **16.67±5.12** | **17.25±5.30** |
| **Model control group** | **15.71±3.34** | **11.63±5.51** | **10.25±3.66**** | **9.50±3.38**** | **9.58±4.50**** | **9.00±5.10**** | **8.96±4.10**** | **9.13±3.93**** |
| **hKGF-2 low-concentration group** | **15.46±3.79** | **11.50±2.59** | **9.83±2.99**** | **9.83±3.35** | **9.88±4.09** | **9.13±4.85** | **13.83±4.54** | **14.38±5.05** |
| **hKGF-2 medium-concentration group** | **16.33±4.09** | **12.04±4.62** | **10.33±3.18**** | **10.75±2.97** | **10.50±4.72** | **11.63±3.43** | **14.46±4.35#** | **15.96±4.20#** |
| **hKGF-2 high-concentration group** | **16.54±4.30** | **12.63±4.06** | **10.50±3.18**** | **11.08±2.46** | **11.21±4.37** | **12.17±3.11** | **15.08±3.07#** | **16.75±4.74##** |
| **Beifushu^{®} eye drop group** | **17.38±4.27** | **11.96±2.84** | **11.04±2.36*** | **11.79±2.47** | **10.75±3.97** | **12.46±4.14** | **14.63±5.52#** | **15.63±6.02#** |
| **Sodium hyaluronate eye drop group** | **15.75±3.12** | **11.50±3.21** | **11.42±2.60*** | **11.04±2.48** | **11.67±4.46** | **12.04±3.26** | **13.33±3.51** | **15.83±5.69#** |

| | | | | | | | | |
|---|---|---|---|---|---|---|---|---|
| Note: ① The time points D0, D7, D15, D19, D23, D26, D30, and D37 in Table 27 represent Day 0, Day 7, Day 15, Day 19, Day 23, Day 26, Day 30, and Day 37 of modeling, respectively. The low-concentration hKGF-2 group refers to the group treated with hKGF-2 at a concentration of 50 µg/ml; the medium-concentration group refers to hKGF-2 at 100 µg/ml; the high-concentration group refers to hKGF-2 at 200 µg/ml. ② Compared with the normal control group: "*" indicates P<0.05, "**" indicates P<0.01; compared with the model control group: "#" indicates P<0.05, "##" indicates P<0.01; compared with the Beifushu^{®} eye drop group: "^" indicates P<0.05, "^^" indicates P<0.01; compared with the sodium hyaluronate eye drop group: "&" indicates P<0.05, "&&" indicates P<0.01. | | | | | | | | |

The results of Table 27 show that:
(1) On modeling days 15, 19, 23, 26, 30, and 37, the basal tear secretion in the model control group was decreased compared with that in the normal control group, and the difference was statistically significant (P<0.01).
(2) On modeling day 30 (day 15 of treatment) and modeling day 37 (the day after treatment ended), the basal tear secretion in the positive control group treated with Beifushu^{®} eye drops was significantly increased compared with that in the model control group, and the difference was statistically significant (P<0.05). On modeling day 37 (the day after treatment ended), the basal tear secretion in the positive control group treated with sodium hyaluronate eye drops was significantly increased compared with that in the model control group, and the difference was statistically significant (P<0.05).
(3) On modeling day 30 (day 15 of treatment) and modeling day 37 (the day after treatment ended), the basal tear secretion in both the medium-concentration hKGF-2 group (100 µg/ml) and the high-concentration hKGF-2 group (200 µg/ml) was significantly increased compared with that in the model control group, and the difference was statistically significant (P<0.01 or P<0.05). The basal tear secretion in the low-concentration hKGF-2 group (50 µg/ml) also showed an increasing trend.
(4) As the treatment duration extended, the basal tear secretion in the low-, medium-, and high-concentration hKGF-2 groups showed an increasing trend.

6.2 The effects of hKGF-2 on tear film stability (BUT) in rabbits are shown in Table 28.

**Table 28: Break-Up Time (BUT) Results Before and After Treatment in Each Group (s) (x±s)**

| **Group** | **D0** | **D7** | **D15** | **D19** | **D23** | **D26** | **D30** | **D37** |
|---|---|---|---|---|---|---|---|---|
| **Normal control group** | **18.12±1.88** | **16.82±3.64** | **18.00±2.17** | **15.79±3.05** | **18.09±1.89** | **14.92±1.95** | **17.21±1.74** | **15.38±2.62** |
| **Model control group** | **18.47±1.79** | **10.91±0.93**** | **7.50±1.29**** | **6.65±0.89**** | **7.62±1.81**** | **6.13±1.15**** | **6.94±0.92**** | **6.17±1.16**** |
| **hKGF-2 low-concentration group** | **19.16±1.35** | **10.78±1.27**** | **7.35±1.23**** | **6.72±0.64** | **8.69±2.13&** | **7.17±1.29^^&&** | **10.21±2.23#** | **8.92±1.81##** |
| **hKGF-2 medium-concentration group** | **17.78±1.13** | **11.19±1.99**** | **7.04±1.11**** | **6.73±1.26** | **10.94±1.84#** | **11.19±2.43##** | **10.42±2.11##** | **9.46±0.99##** |
| **hKGF-2 high-concentration group** | **17.99±2.12** | **10.93±1.27**** | **7.35±1.38**** | **6.35±0.95** | **9.34±2.59&** | **12.16±2.32##** | **10.92±2.92##** | **11.58±1.74##** |
| **Beifushu^{®} eye groupdrop** | **18.63±1.78** | **12.00±1.83**** | **7.28±1.26**** | **7.09±1.38** | **9.88±2.80** | **11.40±1.78##** | **9.83±2.85#** | **9.42±1.54##** |
| **hyaluronate eye Sodium groupdrop** | **17.90±2.80** | **11.35±1.55**** | **7.68±1.56**** | **6.82±1.68** | **12.21±2.57##** | **10.75±1.04##** | **9.96±2.89#** | **9.88±2.32##** |

| | | | | | | | | |
|---|---|---|---|---|---|---|---|---|
| Note: ① The time points D0, D7, D15, D19, D23, D26, D30, and D37 in Table 28 correspond to Day 0, Day 7, Day 15, Day 19, Day 23, Day 26, Day 30, and Day 37 of the modeling period, respectively. The low-concentration group of hKGF-2 refers to hKGF-2 at a concentration of 50 µg/ml, the medium-concentration group refers to 100 µg/ml, and the high-concentration group refers to 200 µg/ml. ② Compared with the normal control group, "*" indicates P<0.05 and "**" indicates P<0.01; compared with the model control group, "#" indicates P<0.05 and "##" indicates P<0.01; compared with the Beifushu^{®} eye drop group, "^" indicates P<0.05 and "^^" indicates P<0.01; compared with the sodium hyaluronate eye drop group, "&" indicates P<0.05 and "&&" indicates P<0.01. | | | | | | | | |

The results of Table 28 show that:
(1) On modeling days 7, 15, 19, 23, 26, 30, and 37, the BUT values in the model control group were decreased compared with those in the normal control group, and the differences were statistically significant (P<0.01).
(2) On modeling day 26 (day 11 of treatment), modeling day 30 (day 15 of treatment), and modeling day 37 (the day after treatment ended), the BUT values in the positive control group treated with Beifushu^{®} eye drops were significantly increased compared with those in the model control group, and the differences were statistically significant (P<0.01 or P<0.05). On modeling day 23 (day 8 of treatment), modeling day 26 (day 11 of treatment), modeling day 30 (day 15 of treatment), and modeling day 37 (the day after treatment ended), the BUT values in the positive control group treated with sodium hyaluronate eye drops were significantly increased compared with those in the model control group, and the differences were statistically significant (P<0.01 or P<0.05).
(3) On modeling day 30 (day 15 of treatment) and modeling day 37 (the day after treatment ended), the BUT values in the low-concentration hKGF-2 group were significantly increased compared with those in the model control group, and the differences were statistically significant (P<0.01 or P<0.05). On modeling day 23 (day 8 of treatment), modeling day 26 (day 11 of treatment), modeling day 30 (day 15 of treatment), and modeling day 37 (the day after treatment ended), the BUT values in the medium-concentration hKGF-2 group were significantly increased compared with those in the model control group, and the differences were statistically significant (P<0.01 or P<0.05). On modeling day 26 (day 11 of treatment), modeling day 30 (day 15 of treatment), and modeling day 37 (the day after treatment ended), the BUT values in the high-concentration hKGF-2 group were significantly increased compared with those in the model control group, and the differences were statistically significant (P<0.01).
(4) As the treatment duration extended, the BUT values in the low-, medium-, and high-concentration hKGF-2 groups showed an increasing trend.

6.3 The effects of hKGF-2 on corneal fluorescein staining scores are shown in Table 29.

**Table 29: Corneal Fluorescein Staining Scores Before and After Treatment in Each Group (x±s)**

| **Group** | **D0** | **D7** | **D15** | **D19** | **D23** | **D26** | **D30** | **D37** |
|---|---|---|---|---|---|---|---|---|
| **Normal control group** | **0.04±0.14** | **0.13±0.23** | **0.08±0.29** | **0.13±0.31** | **0.21±0.33** | **0.08±0.19** | **0.13±0.23** | **0.13±0.31** |
| **Model control group** | **0.17±0.33** | **1.83±1.03**** | **6.25±1.59**** | **5.79±1.29**** | **4.13±1.64**** | **4.21±1.54**** | **4.92±1.08**** | **3.96±1.47**** |
| **hKGF-2 low-concentra tion group** | **0.17±0.44** | **1.67±0.61**** | **5.75±1.25**** | **5.21±1.57** | **2.19±1.22#** | **2.79±1.03#** | **2.92±0.939##** | **2.50±0.83#** |
| **hKGF-2 medium-concentra tion group** | **0.04±0.14** | **1.92±0.73**** | **4.92±2.10**** | **5.46±1.25** | **2.38±1.03##** | **2.13±0.93##** | **2.71±1.27##** | **2.46±1.32#** |
| **hKGF-2 high-concentra tion group** | **0.04±0.14** | **1.58±0.63**** | **5.83±1.75**** | **4.92±0.90** | **2.25±0.69##** | **1.75±0.84##** | **2.54±0.99##** | **2.13±1.03##** |
| **Beifushu ^{®} eye drop group** | **0.17±0.33** | **1.63±0.71**** | **6.29±1.94**** | **5.17±1.17** | **3.13±1.09** | **2.17±0.86##** | **3.13±1.32##** | **2.54±1.42#** |
| **Sodium hyaluron ate eye drop group** | **0.08±0.29** | **1.63±0.93**** | **5.71±1.99**** | **4.71±0.75** | **2.41±0.73##** | **2.33±0.96##** | **2.88±1.77##** | **2.33±0.81##** |

| | | | | | | | | |
|---|---|---|---|---|---|---|---|---|
| Note: ① In Table 29, D0, D7, D15, D19, D23, D26, D30, and D37 represent Day 0, Day 7, Day 15, Day 19, Day 23, Day 26, Day 30, and Day 37 of modeling, respectively. The low-concentration group of hKGF-2 refers to the group treated with hKGF-2 at a concentration of 50 µg/ml; the medium-concentration group refers to the group treated with hKGF-2 at 100 µg/ml; and the high-concentration group refers to the group treated with hKGF-2 at 200 µg/ml. ② Compared with the normal control group, "*" indicates P<0.05 and "**" indicates P<0.01; compared with the model control group, "#" indicates P<0.05 and "##" indicates P<0.01; compared with the Beifushu^{®} eye drop group, "^" indicates P<0.05 and "^^" indicates P<0.01; compared with the sodium hyaluronate eye drop group, "&" indicates P<0.05 and "&&" indicates P<0.01. | | | | | | | | |

The results of Table 29 show that:
(1) On modeling days 7, 15, 19, 23, 26, 30, and 37, the corneal fluorescein staining scores in the model control group were significantly increased compared with those in the normal control group, and the differences were statistically significant (P<0.01).
(2) On modeling day 26 (day 11 of treatment), modeling day 30 (day 15 of treatment), and modeling day 37 (the day after treatment ended), the corneal fluorescein staining scores in the positive control group treated with Beifushu^{®} eye drops were significantly decreased compared with those in the model control group, and the differences were statistically significant (P<0.01 or P<0.05). On modeling day 23 (day 8 of treatment), modeling day 26 (day 11 of treatment), modeling day 30 (day 15 of treatment), and modeling day 37 (the day after treatment ended), the corneal fluorescein staining scores in the positive control group treated with sodium hyaluronate eye drops were significantly decreased compared with those in the model control group, and the differences were statistically significant (P<0.01).
(3) On modeling day 23 (day 8 of treatment), modeling day 26 (day 11 of treatment), modeling day 30 (day 15 of treatment), and modeling day 37 (the day after treatment ended), the corneal fluorescein staining scores in the low-, medium-, and high-concentration hKGF-2 groups were significantly decreased compared with those in the model control group, and the differences were statistically significant (P<0.01 or P<0.05).

The results of the Schirmer I test, tear film break-up time (BUT) test, and corneal fluorescein staining score obtained in the present embodiment demonstrated that the model control group exhibited a significantly lower baseline tear secretion volume and shorter BUT values compared with the normal control group, along with a significantly higher corneal fluorescein staining score, indicating more severe pathological changes. Compared with the model control group, both positive control groups (Beifushu^{®} eye drops and sodium hyaluronate eye drops) effectively alleviated dry eye symptoms in rabbits.

Compared with the model control group, following 21 days of treatment, the baseline tear secretion volume and BUT values of rabbits in the low-, medium-, and high-concentration hKGF-2 groups were significantly increased, while the corneal fluorescein staining scores were significantly reduced, suggesting that all tested concentrations of hKGF-2 were capable of improving dry eye symptoms in rabbits.

6.4 The effects of hKGF-2 on corneal pathological results are shown in Table 30.

**Table 30: Number of Cases and Severity of Lesions in the Cornea and Conjunctiva**

| **Histopathological Examination** | | | **Normal control group (Group A)** | **Model control group (Group B)** | **hKGF-2 low-concentrati on group (Group C)** | **hKGF-2 medium-concentration group (Group D)** | **hKGF-2 high-concentration group (Group E)** | **Beifushu^{®} eye drop group (Group F)** | **Sodium hyaluronat e eye drop group (Group G)** |
|---|---|---|---|---|---|---|---|---|---|
| **Cornea** | **Number of Subjects** | | **12** | **12** | **12** | **12** | **12** | **12** | **12** |
| **- Inflammatory cell infiltration** | **Severity:** | **1** | **0** | **5** | **7** | **4** | **6** | **10** | **7** |
| | | **2** | **0** | **0** | **0** | **1** | **1** | **0** | **0** |
| | | **3** | **0** | **1** | **0** | **0** | **0** | **0** | **0** |
| | | **4** | **0** | **1** | **0** | **0** | **0** | **0** | **0** |
| | **Total Number of Cases** | | **0** | **7** | **6** | **5** | **7** | **9** | **7** |
| **- Epithelial atrophy** | **Severity:** | **1** | **0** | **4** | **4** | **3** | **2** | **2** | **3** |
| | | **2** | **0** | **3** | **2** | **2** | **3** | **2** | **2** |
| | | **3** | **0** | **2** | **1** | **0** | **0** | **2** | **1** |
| | **Total Number of Cases** | | **0** | **9** | **7** | **6** | **5** | **6** | **6** |
| **- Neovascularization** | **Severity:** | **1** | **0** | **6** | **6** | **6** | **5** | **7** | **3** |
| | | **2** | **0** | **2** | **2** | **1** | **1** | **1** | **3** |
| | | **3** | **0** | **1** | **0** | **0** | **0** | **1** | **0** |
| | | **4** | **0** | **1** | **0** | **0** | **0** | **0** | **0** |
| | **Total Number of Cases** | | **0** | **10** | **8** | **7** | **6** | **9** | **6** |
| **Conjunctiva** | **Number Subjectsof** | | **12** | **12** | **12** | **12** | **12** | **12** | **12** |
| **- Goblet reductioncell** | **Severity:** | **1** | **0** | **1** | **5** | **2** | **0** | **2** | **4** |
| | | **2** | **0** | **1** | **3** | **3** | **3** | **4** | **4** |
| | | **3** | **0** | **8** | **4** | **6** | **3** | **3** | **1** |
| | **Total of CasesNumber** | | **0** | **10** | **12** | **11** | **6** | **9** | **9** |

| | | | | | | | | | |
|---|---|---|---|---|---|---|---|---|---|
| Severity Grades: 1 = Slight; 2 = Mild; 3 = Moderate; 4 = Severe. | | | | | | | | | |

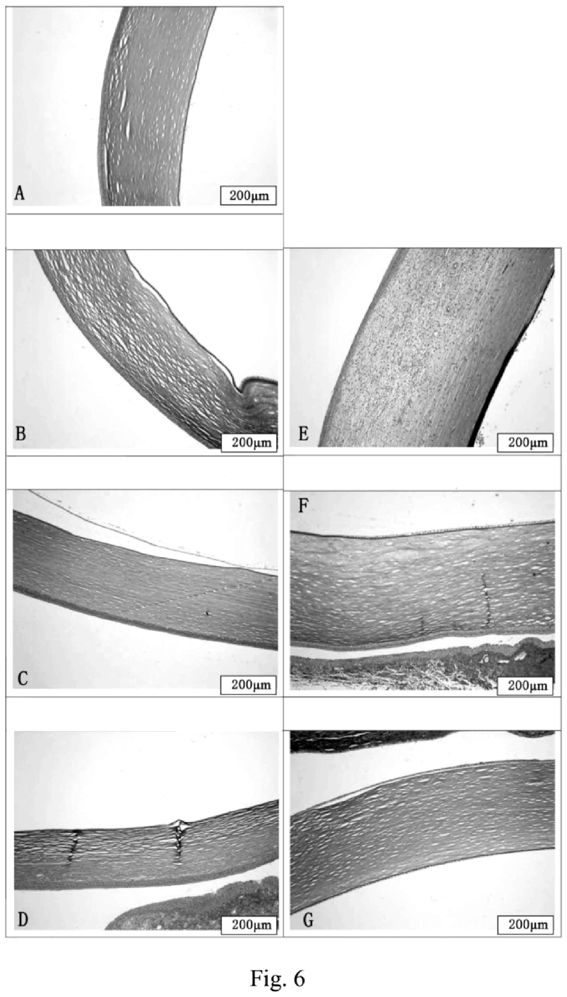

Fig. 6 shows the histopathological results of the effects of hKGF-2 on the cornea (HE staining). All images were obtained at 100× magnification, scale bar = 200 µm.

Fig. 6A illustrates the normal corneal structure of a rabbit in the normal control group (Animal ID: A-12, female). Fig. 6B shows focal corneal epithelial atrophy in a rabbit of the low-concentration hKGF-2 group (Animal ID: C-1, male). Fig. 6C depicts a normal corneal structure in a rabbit of the high-concentration hKGF-2 group (Animal ID: E-5, male). Fig. 6D presents neovascularization in the stromal layer and inflammatory cell infiltration in the corneal epithelium of a rabbit in the sodium hyaluronate eye drop group (Animal ID: G-7, male). Fig. 6E reveals focal epithelial atrophy, neovascularization in the stromal layer, and extensive inflammatory cell infiltration in the model control group (Animal ID: B-5, male). Fig. 6F shows inflammatory cell infiltration in the corneal epithelium of a rabbit in the medium-concentration hKGF-2 group (Animal ID: D-8, female). Fig. 6G illustrates focal epithelial atrophy in a rabbit of the Beifushu^{®} eye drop group (Animal ID: F-3, male).

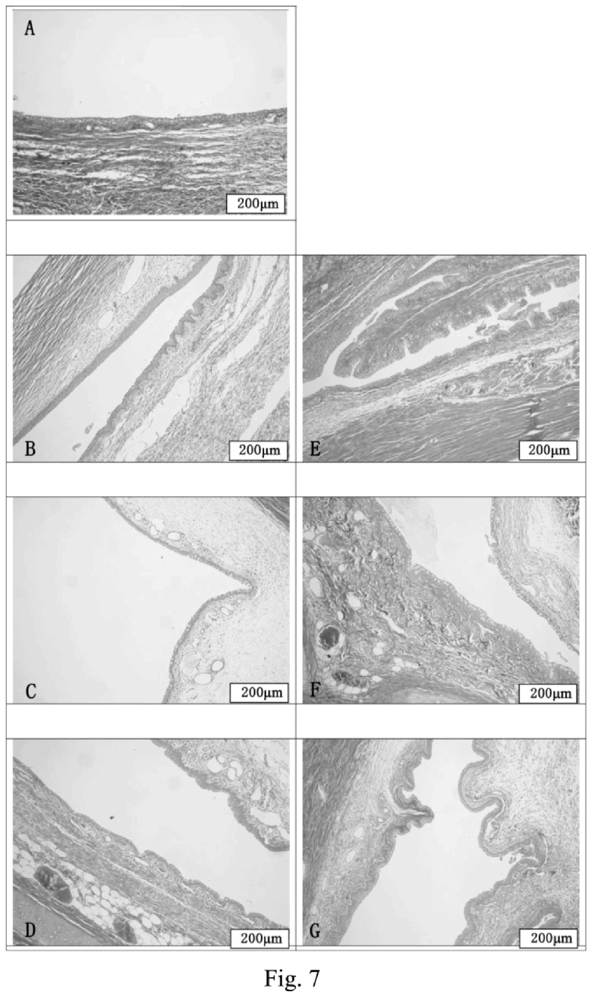

Fig. 7 shows the histopathological results of the effects of hKGF-2 on the conjunctiva (HE staining). All images were obtained at 100× magnification, scale bar = 200 µm.

Fig. 7A illustrates the normal conjunctival structure of a rabbit in the normal control group (Animal ID: A-3, male). Fig. 7B shows a marked reduction in conjunctival epithelial goblet cells in a rabbit of the model control group (Animal ID: B-2, male). Fig. 7C presents a mild reduction in conjunctival epithelial goblet cells in a rabbit of the medium-concentration hKGF-2 group (Animal ID: D-10, female). Fig. 7D shows a reduction in conjunctival epithelial goblet cells in a rabbit of the Beifushu^{®} eye drop group (Animal ID: F-5, male). Fig. 7E illustrates a mild reduction in conjunctival epithelial goblet cells in a rabbit of the low-concentration hKGF-2 group (Animal ID: C-7, male). Fig. 7F depicts a normal conjunctival structure in a rabbit of the high-concentration hKGF-2 group (Animal ID: E-10, female). Fig. 7G shows a reduction in conjunctival epithelial goblet cells in a rabbit of the sodium hyaluronate eye drop group (Animal ID: G-2, male).

The results of Table 30, Fig. 6, and Fig. 7 indicate that, compared with the normal control group, rabbits in the model control group exhibited corneal epithelial atrophy, marked thinning of the epithelial cell layer, disorganized cellular arrangement, and loosened collagen fibers in the stromal layer. Additionally, fibroblast proliferation and neovascularization were observed, accompanied by inflammatory cell infiltration (Fig. 6). The conjunctival epithelial goblet cells in the model control group were also reduced in number (Fig. 7).

After 21 days of treatment, compared with the model control group, the low-, medium-, and high-concentration hKGF-2 eye drop groups, the Beifushu^{®} eye drop group, and the sodium hyaluronate eye drop group all exhibited improvements in corneal epithelial atrophy and inflammatory cell infiltration. The degree and incidence of goblet cell reduction in the conjunctival epithelium were also alleviated. Among these, the low-, medium-, and high-concentration hKGF-2 eye drop groups demonstrated superior corneal tissue repair compared with the Beifushu^{®} and sodium hyaluronate eye drop groups (Fig. 6 and Fig. 7). Notably, the high-concentration hKGF-2 eye drop group showed greater recovery in both corneal damage and conjunctival lesions than the other groups.

Therefore, the low-, medium-, and high-concentration hKGF-2 eye drop groups, the Beifushu^{®} eye drop group, and the sodium hyaluronate eye drop group were all capable of alleviating pathological damage to the cornea and conjunctiva. Among these, the low-, medium-, and high-concentration hKGF-2 eye drop groups demonstrated superior efficacy in improving corneal pathology compared with the Beifushu^{®} and sodium hyaluronate eye drop groups.

Compared with the blank control group, the pathological severity in the model control group was greater. In comparison with the model control group, on treatment day 21, the low-, medium-, and high-concentration hKGF-2 eye drop groups showed a significant increase in tear film break-up time, a significant decrease in corneal fluorescein staining scores, and alleviation of corneal pathological severity, suggesting that all three concentrations were effective in improving dry eye symptoms in rabbits.

Therefore, under the experimental conditions of the present embodiment, low-, medium-, and high-concentration (low-, medium-, and high-dose) hKGF-2 were able to alleviate dry eye symptoms in rabbits induced by benzalkonium chloride.

It should be noted that the above-mentioned embodiments are exemplary. Those skilled in the art, inspired by the present disclosure, may devise various solutions within the scope and protection of the present disclosure. Furthermore, those skilled in the art will recognize that the specification and accompanying drawings provided herein are illustrative and form no limitation to any of the appended claims. The protection scope of the present application is defined by the appended claims and their equivalents. The specification provided herein encompasses multiple inventive concepts, with terms like "preferably" or "according to a preferred embodiment" each indicating an independent inventive concept disclosed in the respective paragraph. The applicant reserves the right to file divisional applications for each inventive concept. Throughout the specification, features introduced by the term "preferably" are merely optional and should not be construed as mandatory; accordingly, the applicant reserves the right to abandon or delete such preferred features at any time.

## Claims

1. A composition for use in treatment of ocular disorders, the composition comprising human keratinocyte growth factor-2 (hKGF-2) and a stabilizing agent, wherein the stabilizing agent comprises one or more of:
1) 0.1% to 2% (w/v) mannitol or 0.01% to 0.05% (w/v) sodium hyaluronate;
2) 0.1% to 2% (w/v) glycerol;
3) 0.1% to 2% (w/v) trehalose or 0.5% (w/v) human serum albumin; and
4) 0.1% to 2% (w/v) lysine hydrochloride.

2. The composition of claim 1, wherein the composition comprises mannitol at a concentration selected from 0.5% or from 1% to 2% (w/v), preferably 0.5% or 1.6% (w/v).

3. The composition of claim 1 or 2, wherein the composition comprises glycerol at a concentration of 0.5% to 2% (w/v), preferably 1%, 1.5% or 0.5% (w/v).

4. The composition of claim 3, wherein the composition comprises trehalose at a concentration of 0.5% to 2% (w/v), preferably 2% (w/v).

5. The composition of claim 4, wherein the composition comprises lysine hydrochloride at a concentration of 0.1% to 1% (w/v), preferably 0.1% or 0.5% (w/v).

6. The composition of claim 5, wherein the composition further comprises a surfactant comprising poloxamer 188 at a concentration of 0.01% to 0.5% (w/v), preferably 0.01% to 0.1% (w/v), and more preferably 0.05% or 0.01% (w/v).

7. The composition of claim 6, further comprising a pH regulator comprising citric acid, sodium citrate, or a combination thereof.

8. The composition of claim 7, further comprising a solvent, which may be water for injection.

9. The composition of claim 7, wherein the composition has a pH of 6.0 to 7.0, preferably 6.0 to 6.6.

10. Use of the composition according to any one of claims 1 to 9 in the preparation of a medicament for the treatment of dry eye disease.

11. Use of the composition according to any one of claims 1 to 9 in the preparation of a medicament for the treatment of benzalkonium chloride-induced dry eye disease.

12. Use of the composition according to any one of claims 1 to 9 for improving tear film stability, promoting the repair of corneal injury, and/or reducing the severity of conjunctival lesions.

13. A container comprising the composition according to any one of claims 1 to 9, wherein the container is preferably an eye drop vial.

14. A kit comprising the container of claim 13 and instructions and/or a label for the administration and use of the composition according to any one of claims 1 to 9.

15. A method for preparing a composition for use in treatment of ocular disorders, the method comprising steps of:
preparing Solution A comprising glycerol, mannitol, lysine hydrochloride, trehalose, poloxamer 188, citric acid, and sodium citrate;
preparing Solution B comprising a prescribed amount of a stock solution of human keratinocyte growth factor-2 (hKGF-2) protein; and
mixing Solution A and Solution B to obtain the composition.
